(19) Europäisches Patentamt
European Patent Office
Office européen des brevets

(11) EP 4 317 165 B1

(12) **EUROPEAN PATENT SPECIFICATION**

(45) Date of publication and mention of the grant of the patent:
**06.05.2026 Bulletin 2026/19**

(21) Application number: **22775737.4**

(22) Date of filing: **24.03.2022**

(51) International Patent Classification (IPC):
***C07F 5/02*** (2006.01) ***A61P 35/00*** (2006.01)
***C07B 59/00*** (2006.01) ***C07C 227/16*** (2006.01)
***C07C 229/36*** (2006.01) ***C07C 269/06*** (2006.01)

(52) Cooperative Patent Classification (CPC):
(C-Sets available)
**C07F 5/025; A61P 35/00; C07B 59/001; C07C 227/16; C07C 269/06;** C07B 2200/07; Y02P 20/55 (Cont.)

(86) International application number:
**PCT/JP2022/013808**

(87) International publication number:
**WO 2022/202956 (29.09.2022 Gazette 2022/39)**

(54) **BORONIC ACID COMPOUND AND METHOD FOR PRODUCING SAME**

BORSÄUREVERBINDUNG UND VERFAHREN ZUR HERSTELLUNG DAVON

COMPOSÉ D'ACIDE BORONIQUE ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2021 JP 2021052352**

(43) Date of publication of application:
**07.02.2024 Bulletin 2024/06**

(73) Proprietor: **OSAKA UNIVERSITY**
**Suita-shi**
**Osaka 565-0871 (JP)**

(72) Inventors:
- **SHIRAKAMI, Yoshifumi**
  **Suita-shi, Osaka 565-0871 (JP)**
- **KANEDA, Kazuko**
  **Suita-shi, Osaka 565-0871 (JP)**
- **KADONAGA, Yuichiro**
  **Suita-shi, Osaka 565-0871 (JP)**
- **WATABE, Tadashi**
  **Suita-shi, Osaka 565-0871 (JP)**
- **TOYOSHIMA, Atsushi**
  **Suita-shi, Osaka 565-0871 (JP)**
- **FUKASE, Koichi**
  **Suita-shi, Osaka 565-0871 (JP)**
- **SHINOHARA, Atsushi**
  **Suita-shi, Osaka 565-0871 (JP)**
- **YAMANAKA, Toshio**
  **Inashiki-gun, Ibaraki 300-0326 (JP)**
- **KONDOH, Yutaka**
  **Inashiki-gun, Ibaraki 300-0326 (JP)**

(74) Representative: **Weickmann & Weickmann PartmbB**
**Postfach 860 820**
**81635 München (DE)**

(56) References cited:
**WO-A1-2019/027059 WO-A1-2019/176505**
**WO-A1-2020/180390 US-A1- 2021 000 988**

- **GHOSH SAMIR ET AL: "Formal synthesis of piperazinomycin, a novel antifungal antibiotic", vol. 2009, no. 7, 1 January 2009 (2009-01-01), pages 72 - 78, XP055969045, Retrieved from the Internet <URL:http://www.arkat-usa.org/get-file/28242/> DOI: 10.3998/ark.5550190.0010.707**
- **DATABASE REAXYS [online] 1 January 1979 (1979-01-01), VISSER: "The preparation and stability of astatotyrosine and astato-iodotyrosine", XP093243839, Database accession no. Rx-ID: 7313711**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 317 165 B1

- **VISSER G W M ET AL: "The preparation and stability of astatotyrosine and astato-iodotyrosine", INTERNATIONAL JOURNAL OF APPLIED RADIATION AND ISOTOPS, PERGAMON PRESS, NEW YORK, NY, US, vol. 30, no. 12, 1 December 1979 (1979-12-01), pages 749 - 752, XP022601010, ISSN: 0020-708X, [retrieved on 19791201], DOI: 10.1016/0020-708X(79) 90154-6**
- **DATABASE REAXYS [online] 7 January 2021 (2021-01-07), OSAKA UNIVERSITY: "PHARMACEUTICAL COMPOSITION CONTAINING 211AT-LABELED AMINO ACID DERIVATIVE, AND METHOD FOR PRODUCING SAID PHARMACEUTICAL COMPOSITION - US2021000988 A1", XP093243840, Database accession no. Rx-ID: 56116611**
- **GHOSH SAMIR, KUMAR A.SANJEEV, MEHTA G.N., SOUNDARARAJAN R., SEN SUBHABRATA: "Formal synthesis of piperazinomycin, a novel antifungal antibiotic", ARKIVOC, vol. 2009, no. 7, 1 January 2009 (2009-01-01), pages 72 - 78, XP055969045, DOI: 10.3998/ ark.5550190.0010.707**
- **NG-CHOI ITENG, OLIVERAS ÀNGEL, FELIU LIDIA, PLANAS MARTA: "Solid-phase synthesis of biaryl bicyclic peptides containing a 3-aryltyrosine or a 4-arylphenylalanine moiety", BEILSTEIN JOURNAL OF ORGANIC CHEMISTRY, vol. 15, no. 15, 22 March 2019 (2019-03-22), pages 761 - 768, XP055969047, DOI: 10.3762/bjoc.15.72**
- **NG-CHOI ITENG; OLIVERAS ÀNGEL; PLANAS MARTA; FELIU LIDIA: "Solid-phase synthesis of biaryl cyclic peptides containing a histidine-tyrosine linkage", TETRAHEDRON, ELSEVIER SIENCE PUBLISHERS, AMSTERDAM, NL, vol. 75, no. 18, 1 January 1900 (1900-01-01), AMSTERDAM, NL , pages 2625 - 2636, XP085658921, ISSN: 0040-4020, DOI: 10.1016/j.tet.2019.03.014**


(52) Cooperative Patent Classification (CPC): (Cont.)

C-Sets
**C07C 227/16, C07C 229/36;**
**C07C 269/06, C07C 271/22**

## Description

### Technical Field

The present invention relates to novel boronic acid compounds which are intermediates for producing radiolabeled tyrosine derivatives useful as anti-cancer drugs, and methods for producing the same, and methods for producing radiolabeled tyrosine derivatives using the same.

### Background Art

Radiolabeled tyrosine derivatives such as astato($^{211}$At)-α-methyl-L-tyrosine ($^{211}$At-AAMT) are drugs that are taken up into tumor cells via LAT1 amino acid transporter specifically expressed in tumors, and are expected to be useful as anti-cancer drugs (Patent Document 1).

Patent Document 1 discloses that $^{211}$At-AAMT is conventionally produced by dissolving α-methyl-L-tyrosine (AMT) in sulfuric acid, adding mercury sulfate to the solution to introduce mercury on the benzene ring, and then subjecting the resulting compound to an astatine exchange reaction (hereinafter to be also referred to as the mercury method). Since this method requires use of mercury, which is a hazardous substance, it cannot be said to be a method suitable for the production of pharmaceuticals in terms of safety. In addition, the above method is not suitable for industrial production because the synthetic yield varies relatively greatly between production batches. Furthermore, iodine-substituted compounds and halogen-disubstituted compounds are produced as byproducts, which poses a problem in terms of purity. It has also been reported that the produced $^{211}$At-AAMT is unstable (Non-Patent Document 1).

In addition, it is generally known that halogen is easily introduced into the 3-position of a tyrosine derivative by halogenation in the presence of an oxidant. However, this method is not effective only for astatine (Non-Patent Document 2).

On the other hand, the present inventors have already reported that a boryl group ($-B(OH)_2$) introduced into an aryl group has excellent astatine-substituting ability (Patent Document 2).

Patent Document 3 discloses borylated amino acid compositions and a method of preparing the same. An examplary compound TLS00192 is based on tyrosine.

Patent Document 4 discloses astato-labeled amino acid derivatives and methods for the preparation thereof.

Non-Patent Document 3 describes the synthesis of piperazinomycin based on intramolecular O-arylation of arylboronic acid with phenol for formation of the macrocyclic biaryl ether.

Non-Patent Document 4 discloses the preparation and stability of astatotyrosine.

### Document List

#### Patent Document

[Patent Document 1] WO 2019/176505
[Patent Document 2] WO 2019/027059
[Patent Document 3] WO 2020/180390
[Patent Document 4] US 2021/000988

#### Non-Patent Document

[Non-Patent Document 1] J Surg Oncol 1988; 37:192-7
[Non-Patent Document 2] Int J Appl Radiat Isotop 1979; 30: 749-52
[Non-Patent Document 3] Ghosh Samir et al., ARKIVOC 2009, no.7, pp. 72-78
[Non-Patent Document 4] Visser et al., Int.J.Appl. Ratiation and Isotops, 1979, 99. 749-752

### Summary of the Invention

#### Problems to be Solved by the Invention

The present invention aims to provide a method for producing radiolabeled tyrosine derivatives with good purity

and stability, by a safe method suitable for industrial production of pharmaceuticals.

**Means of Solving the Problems**

The present inventors have focused on the production method described in Patent Document 2 as a production method that does not require use of hazardous substances such as mercury, and have conducted intensive studies in an attempt to produce tyrosine boronic acid derivatives or its esters as raw materials for the method, which resulted in the completion of the present invention.

Accordingly, the present invention provides the following.

[1] A method for producing a compound represented by Formula (5) or a salt thereof (hereinafter to be also referred to as Compound (5)), comprising the following Steps 1 to 4;

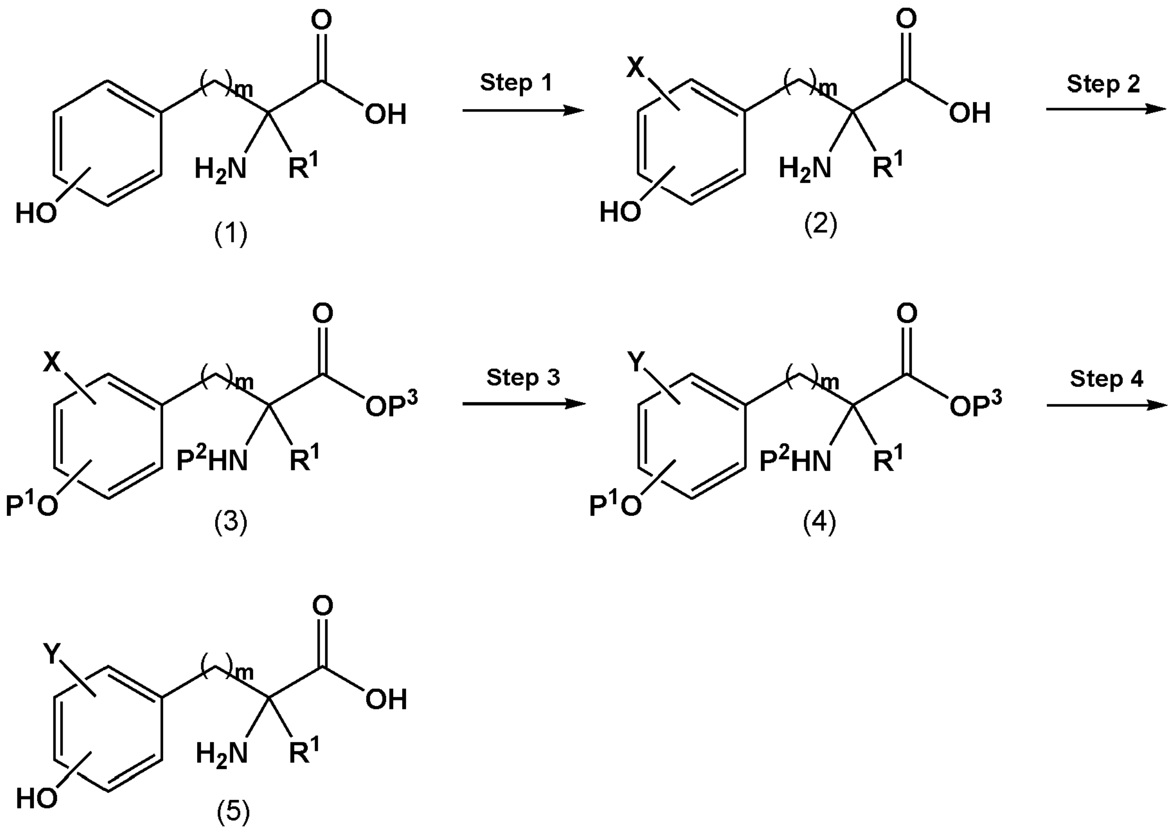


(1) (2) (3) (4) (5)

wherein

$R^1$ is a methyl group;
$P^1$ is an ether-type hydroxy-protecting group;
$P^2$ is an amino-protecting group;
$P^3$ is a carboxy-protecting group;
m is 0, 1 or 2;
X is a halogen atom; and
Y is a boryl group ($-B(OH)_2$) or its ester group,
Step 1: a step of halogenating a compound represented by Formula (1) or a salt thereof (hereinafter to be also referred to as Compound (1)) to obtain a compound represented by Formula (2) or a salt thereof (hereinafter to be also referred to as Compound (2));
Step 2: a step of protecting the amino group and carboxy group of the compound represented by Formula (2) or a salt thereof and protecting the hydroxy group of the compound or salt with an ether-type protecting group to obtain a compound represented by Formula (3) (hereinafter to be also referred to as Compound (3));
Step 3: a step of reacting the compound represented by Formula (3) with a reagent for introducing boronic acid in the presence of a palladium catalyst and a base to obtain a compound represented by Formula (4) (hereinafter to

be also referred to as Compound (4)); and

Step 4: a step of removing the protecting groups for the carboxy group, amino group and hydroxy group of the compound represented by Formula (4) to obtain the compound represented by Formula (5) or a salt thereof.

[2] The method of the above-mentioned [1], wherein $P^1$ is a benzyl group or a p-methoxybenzyl group.

[3] The method of the above-mentioned [1] or [2], wherein $P^3$ is a benzyl group or a $C_{1-2}$ alkyl group.

[4] The method of the above-mentioned [1], wherein $P^1$ and $P^3$ are both benzyl groups.

[5] The method of any one of the above-mentioned [1] to [4], wherein the bonding position of the hydroxy group on the benzene ring in Formula (5) is the 4-position or 3-position.

[6] The method of the above-mentioned [5], wherein the bonding positions of the hydroxy group and the group -Y on the benzene ring in Formula (5) are adjacent to each other.

[7] The method of any one of the above-mentioned [1] to [4], wherein, in Formula (5), the bonding position of the hydroxy group on the benzene ring is the 4-position, and the bonding position of the group -Y on the benzene ring is the 3-position.

[8] The method of any one of the above-mentioned [1] to [7], wherein the palladium catalyst to be used in Step 3 is [1,1'-bis(diphenylphosphino)ferrocene]dichloropalladium(II) ($PdCl_2$(dppf))).

[9] The method of the above-mentioned [8], wherein the reaction of Step 3 is carried out in a sulfoxide solvent or an amide solvent.

[10] The method of the above-mentioned [8], wherein the base to be used in Step 3 is an alkali metal acetate.

[11] The method of any one of the above-mentioned [1] to [10], wherein Y is a boryl group ($-B(OH)_2$) or a 4,4,5,5-tetramethyl-1,3,2-dioxaboran-2-yl group.

[12] Additionally described herein but not claimed is a method for producing a compound represented by Formula (5) or a salt thereof, comprising the following Step 4;

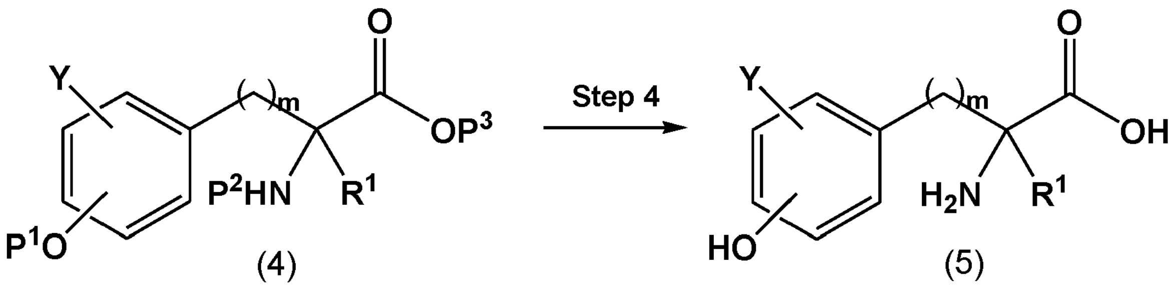


(4) (5)

wherein

$R^1$ is a hydrogen atom or a $C_{1-4}$ alkyl group;

$P^1$ is an ether-type hydroxy-protecting group;

$P^2$ is an amino-protecting group;

$P^3$ is a carboxy-protecting group;

m is 0, 1 or 2; and

Y is a boryl group ($-B(OH)_2$) or its ester group,

Step 4: a step of removing the protecting groups for the carboxy group, amino group and hydroxy group of a compound represented by Formula (4) to obtain the compound represented by Formula (5) or a salt thereof.

[14] Additionally described herein but not claimed is a method for producing a compound represented by Formula (4), comprising the following Step 3;

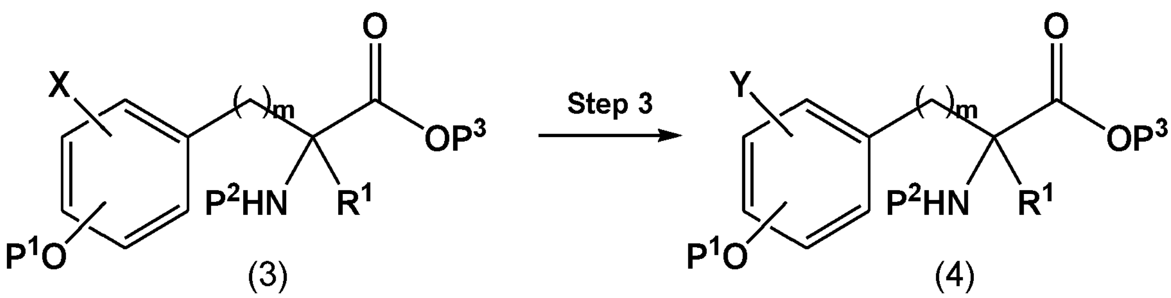


(3) (4)

wherein

$R^1$ is a hydrogen atom or a $C_{1-4}$ alkyl group;
$P^1$ is an ether-type hydroxy-protecting group;
$P^2$ is an amino-protecting group;
$P^3$ is a carboxy-protecting group;
m is 0, 1 or 2;
X is a halogen atom; and
Y is a boryl group (-B(OH)$_2$) or its ester group,
Step 3: a step of reacting a compound represented by Formula (3) with a reagent for introducing boronic acid in the presence of a palladium catalyst and a base to obtain the compound represented by Formula (4).

[15] A compound represented by the following Formula (5a) or a salt thereof (hereinafter to be also referred to as Compound (5a));

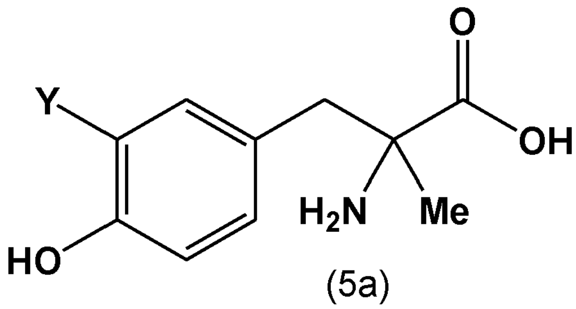

(5a)

wherein Y is a boryl group (-B(OH)$_2$) or its ester group.
[16] A compound represented by the following Formula (4a) (hereinafter to be also referred to as Compound (4a));

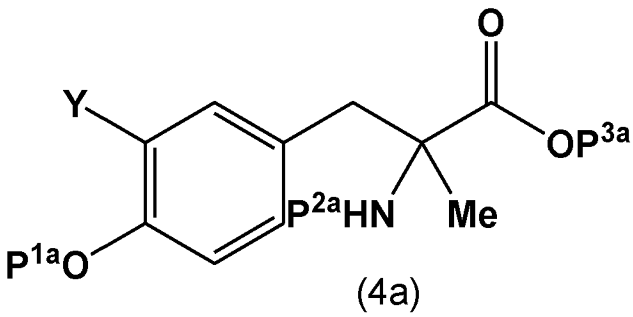

(4a)

wherein

$P^{1a}$ is a benzyl group or a p-methoxybenzyl group;
$P^{2a}$ is a tert-butoxycarbonyl group;
$P^{32}$ is a benzyl group or a $C_{1-2}$ alkyl group; and
Y is a boryl group (-B(OH)$_2$) or its ester group.

[17] A method for producing a radiolabeled compound represented by Formula (6) or a salt thereof (hereinafter to be also referred to as Radiolabeled Compound (6)), comprising the following Step 5;

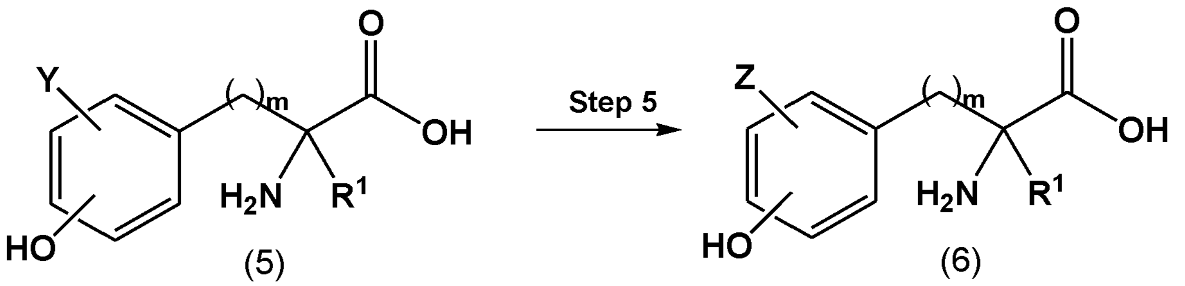

wherein

$R^1$ is a hydrogen atom or a $C_{1-4}$ alkyl group;
m is 0, 1 or 2;
Y is a boryl group (-$B(OH)_2$) or its ester group; and
Z is $^{211}At$, $^{210}At$, $^{123}I$, $^{124}I$, $^{125}I$ or $^{131}I$,
Step 5: a step of reacting a compound represented by Formula (5) or a salt thereof with a radionuclide selected from $^{211}At$, $^{210}At$, $^{123}I$, $^{124}I$, $^{125}I$ and $^{131}I$, in the presence of a reagent selected from an alkali metal iodide, an alkali metal bromide, N-bromosuccinimide, N-chlorosuccinimide, N-iodosuccinimide and hydrogen peroxide, in water to obtain the radiolabeled compound represented by Formula (6) or a salt thereof.

[18] The method of the above-mentioned [17], wherein the compound represented by Formula (5) or a salt thereof is produced by a method as defined in any one of the above-mentioned [1] to [12].
[19] The method of the above-mentioned [17] or [18], wherein the reaction is carried out in an organic solvent-free system.
[20] The method of any one of the above-mentioned [17] to [19], wherein the reaction is carried out within the range of room temperature to 100°C.
[21] The method of any one of the above-mentioned [17] to [20], wherein the radionuclide is $^{211}At$ or $^{131}I$, and the reagent is selected from potassium iodide and N-bromosuccinimide.
[22] The method of any one of the above-mentioned [17] to [21], further comprising a step of purifying the radiolabeled compound represented by Formula (6) or a salt thereof.
[23] The method of any one of the above-mentioned [17] to [22], further comprising a step of stabilizing the radiolabeled compound represented by Formula (6) or a salt thereof by adding ascorbic acid.

**Effect of the Invention**

According to the present invention, tyrosine derivatives into which a boryl group (-$B(OH)_2$) or its ester group is introduced can be obtained, and therefore, the use of the compounds makes it possible to produce radiolabeled tyrosine derivatives with good purity and stability, by a safe method suitable for industrial production of pharmaceuticals without using hazardous substances.

**Brief Description of the Drawings**

[Figure 1]
Figure 1 shows the HPLC chart of 3-(4,4,5,5-tetramethyl-1,3,2-dioxaboran-2-yl)-α-methyl-L-tyrosine hydrochloride (5) obtained in Example 1.
[Figure 2]
Figure 2 shows the HPLC chart of 3-borono-α-methyl-L-tyrosine hydrochloride (5) obtained in Example 2.
[Figure 3]
Figure 3 shows the results of thin-layer chromatography (TLC) analysis of the reaction solution prepared in Example 3.
[Figure 4]
Figure 4 shows the results of thin-layer chromatography (TLC) analysis of the reaction solution prepared in Example 4.
[Figure 5]
Figure 5 shows the results of thin-layer chromatography (TLC) analysis of the reaction solution prepared in Example 5.
[Figure 6]
Figure 6a shows the $^{211}At$-AAMT intracellular uptake (synthesized by the method of the present invention), and Figure

6b shows the $^{211}$At-AAMT intracellular uptake (synthesized by the mercury method).
[Figure 7]
Figure 7 shows cell viability after addition of $^{211}$At-AAMT.

## Description of Embodiments

The present invention is explained in detail in the following.

In the present specification, examples of the "halogen atom" include a fluorine atom, a chlorine atom, a bromine atom and an iodine atom.

In the present specification, examples of the "$C_{1-4}$ alkyl group" include methyl, ethyl, propyl, isopropyl, butyl, isobutyl, sec-butyl and tert-butyl.

In the present specification, examples of the "$C_{1-6}$ alkyl group" include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl and 2-ethylbutyl.

In the present specification, examples of the "ether-type hydroxy-protecting group" include a benzyl group, a p-methoxybenzyl group, a methoxymethyl group, a trimethylsilyl group, a triethylsilyl group, a trityl group, a tertbutyldimethylsilyl group, a tetrahydropyranyl group and the like. Among them, benzyl ether-type hydroxy-protecting groups such as a benzyl group and a p-methoxybenzyl group are preferred.

In the present specification, examples of the "amino-protecting group" include a tert-butoxycarbonyloxy group, a benzyloxycarbonyl group, a 9-fluorenylmethyloxycarbonyl group and the like.

In the present specification, examples of the "carboxy-protecting group" include a benzyl group, a $C_{1-2}$ alkyl group (a methyl group, an ethyl group), a tert-butyl group and the like.

In the present specification, the term "boryl group ($-B(OH)_2$)" is also referred to as a dihydroxyboryl group.

In the present specification, examples of the "ester group of boryl group" include the following groups.

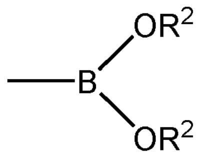
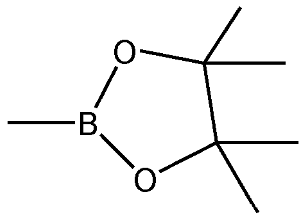
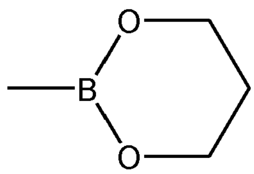
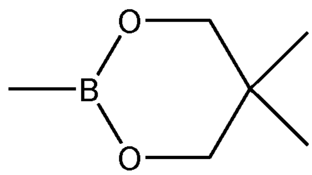

wherein $R^2$ is a $C_{1-6}$ alkyl group.

$R^1$ is a methyl group.

$P^1$ is preferably a benzyl ether-type hydroxy-protecting group, more preferably a benzyl group or a p-methoxybenzyl group, particularly preferably a benzyl group.

$P^2$ is preferably a tert-butoxycarbonyloxy group or a benzyloxycarbonyl group, more preferably a tert-butoxycarbonyloxy group.

$P^3$ is preferably a benzyl group or a $C_{1-2}$ alkyl group (a methyl group, an ethyl group), more preferably a benzyl group.

m is preferably 1.

X is preferably an iodine atom or a bromine atom, particularly preferably an iodine atom.

Y is preferably a boryl group ($-B(OH)_2$) or a 4,4,5,5-tetramethyl-1,3,2-dioxaboran-2-yl group.

In the present specification, when Compound (1), Compound (2), Compound (5) and Radiolabeled Compound (6) are each a salt, examples of such salt include metal salts (e.g., alkali metal salts such as sodium salt and potassium salt; alkaline-earth metal salts such as calcium salt, magnesium salt and barium salt), ammonium salts, salts with organic bases (e.g., trimethylamine, triethylamine, pyridine, picoline, 2,6-lutidine), salts with inorganic acids (e.g., hydrochloric acid, hydrobromic acid, nitric acid, sulfuric acid), salts with organic acids (e.g., formic acid, acetic acid, trifluoroacetic acid, phthalic acid, fumaric acid, oxalic acid, tartaric acid, maleic acid, citric acid, succinic acid, malic acid), and the like.

In the present invention, a method for producing Compound (5) comprises the following Steps 1 to 4.

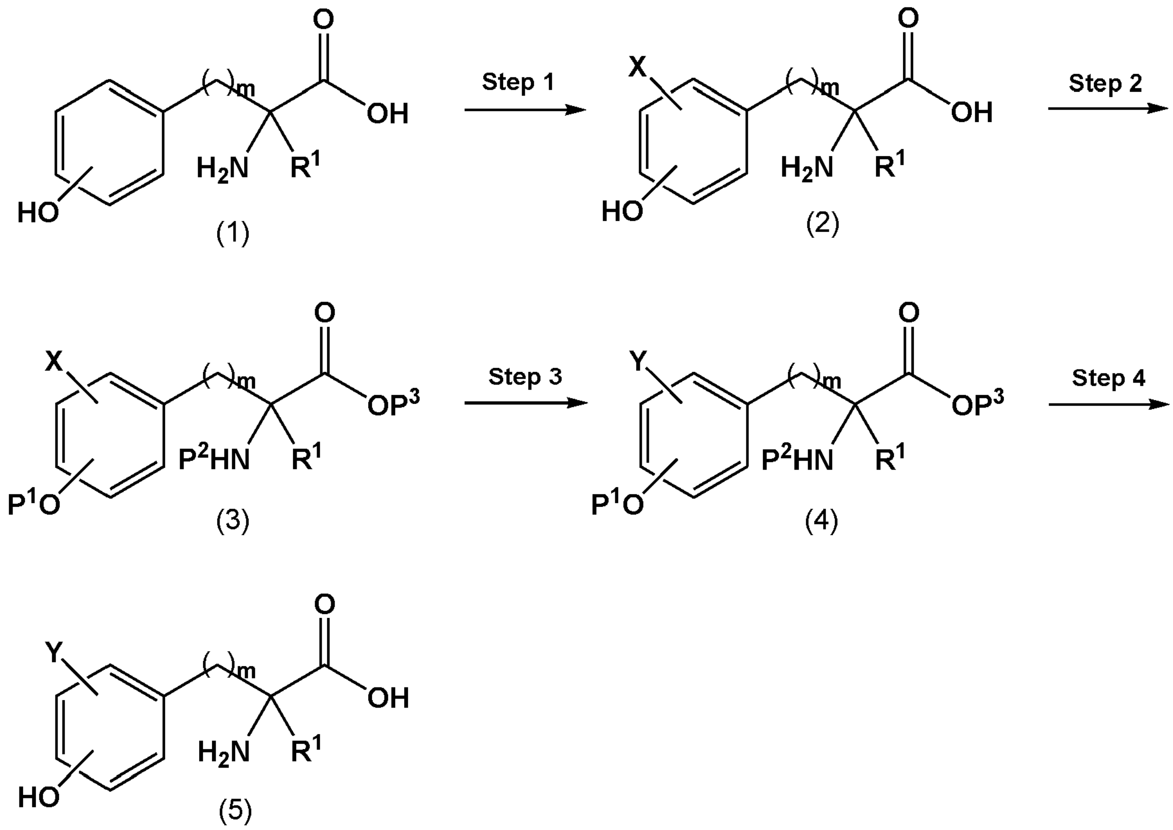


(1) (2) (3) (4) (5)

Step 1 is a step of halogenating Compound (1) to obtain Compound (2).

The halogenation can be carried out by reacting Compound (1) with a halogenating agent.

Examples of Compound (1) include tyrosine, α-methyltyrosine, m-tyrosine, α-methyl-m-tyrosine and the like. Compound (1) may be L-form, D-form or DL-form. Among them, tyrosine and α-methyltyrosine are preferably used, and α-methyltyrosine is particularly preferably used. Compound (1) may be a commercially available product.

The halogenating agent to be used is preferably an iodinating agent or a brominating agent, particularly preferably an iodinating agent.

Examples of the iodinating agent include iodine, N-iodosuccinimide and the like.

Examples of the brominating agent include bromine, N-bromosuccinimide and the like.

The amount of the halogenating agent to be used is generally 1 to 5 mol, preferably 1 to 2 mol, per 1 mol of Compound (1).

When the halogenating agent is iodine, the reaction is carried out in the presence of potassium iodide and conc. ammonia (28%). The amount of the potassium iodide to be used is generally 0.5 to 5 mol, preferably 1 to 2 mol, per 1 mol of the iodine, and the amount of the conc. ammonia to be used is generally 5 to 200 mol, preferably 20 to 50 mol, per 1 mol of the iodine.

The reaction is carried out generally in a solvent. The solvent to be used in the present invention is not particularly limited as long as it does not adversely affect the reaction. Examples thereof include water; alcohol solvents such as ethanol, methanol and isopropanol; halogen solvents such as carbon tetrachloride, and the like. These may be used in combination of two or more. Among them, water is preferably used.

The amount of the solvent to be used is generally 0.1 to 100-fold in volume relative to Compound (1).

When the halogenating agent is iodine, the reaction is carried out preferably, for example, by adding (preferably dropwise) a mixture of Compound (1) and conc. ammonia (and solvent if necessary) to a mixture of iodine, potassium iodide and solvent.

The reaction is carried out generally within the range of -100 to 20°C, preferably -20 to 10°C. The reaction time varies depending on the reaction temperature, and it is generally about 30 min to about 24 hr, preferably about 1 to about 12 hr.

The completion of the reaction can be confirmed by thin-layer chromatography, liquid chromatography and the like.

After the completion of the reaction, Compound (2) can be isolated and/or purified from the reaction mixture by separation means such as concentration, crystallization, recrystallization, distillation, solvent extraction, fractional distillation and chromatography, according to a conventional method.

By this reaction, a halogen is introduced into the 3-position of the benzene ring in the case of tyrosine and α-methyltyrosine (both of which have a hydroxy group at the 4-position of the benzene ring), and into the 4-position or 6-position of the benzene ring in the case of m-tyrosine and α-methyl-m-tyrosine (both of which have a hydroxy group at the 3-position of the benzene ring).

Step 2 is a step of protecting the amino group and carboxy group of Compound (2), and protecting the hydroxy group with an ether-type protecting group to obtain Compound (3).

The present inventors initially attempted to introduce a boryl group or its ester group into Compound (2) by utilizing a well-known reaction of a halogen compound with a reagent for introducing boronic acid. However, it was unexpectedly found that the reaction hardly proceeded. Predicting that the hydroxy group on the benzene ring might be the cause, they attempted to protect the hydroxy group with various protective groups before introducing the boryl group or its ester group. As a result, it was unexpectedly found that, depending on the type of protecting group, the introduction reaction of the boryl group or its ester group may be difficult to proceed.

The present inventors have conducted intensive studies on hydroxy-protecting groups in order to solve such hitherto unknown problems specific to tyrosine derivatives, that is, specific to benzene rings having a hydroxy group, and found for the first time that, by adopting a specific protecting group (an ether-type protecting group, especially a benzyl ether-type protecting group), a boryl group or its ester group can be introduced into the benzene ring. Thus, in the present invention, the selection of the hydroxy-protecting group for Compound (2) is an important key in the introduction of a boryl group or its ester group.

In the present invention, the hydroxy-protecting group is an ether-type protecting group ($P^1$). As long as the hydroxy-protecting group is an ether-type protecting group ($P^1$), a boryl group or its ester group can be introduced into the benzene ring. On the other hand, when the hydroxy-protecting group is an ester-type protecting group such as an acetyl group, a benzoyl group and a pivaloyl group, the introduction reaction of a boryl group or its ester group hardly proceeds.

In terms of the introduction of a boryl group or its ester group in a good yield, the ether-type hydroxy-protecting group ($P^1$) is preferably a benzyl ether-type protecting group, more preferably a benzyl group or a p-methoxybenzyl group, particularly preferably a benzyl group. Since a benzyl group and a p-methoxybenzyl group have not much steric hinderance, and thus a boryl group or its ester group can be introduced in a high yield.

The present inventors also found that the introduced boryl group or its ester group may be unexpectedly eliminated under conditions of strong acids such as highly concentrated hydrochloric acid, nitric acid, sulfuric acid, boron tribromide and boron trifluoride in the deprotection step after introduction. Such elimination is usually difficult to occur in benzene rings having no hydroxy group, and is a problem specific to benzene rings having a hydroxy group.

The present inventors have further conducted intensive studies on hydroxy-protecting groups in order to solve such hitherto unknown problems specific to tyrosine derivatives, that is, specific to benzene rings having a hydroxy group, and found for the first time that, by adopting a benzyl ether-type protecting group (particularly a benzyl group or a p-methoxybenzyl group), the subsequent deprotection step can be carried out under milder conditions (e.g., catalytic hydrogenation). A p-methoxybenzyl group can also be removed with an oxidant such as 2,3-dichloro-5,6-dicyano-p-benzoquinone, trifluoroacetic acid, and relatively low-concentrated hydrochloric acid. The present inventors also found that, by adopting such deprotection method, the elimination of the boryl group or its ester group is difficult to occur.

Thus, in the present invention, the selection of the hydroxy-protecting group for Compound (2) is an important key in the elimination of the boryl group or its ester group.

Moreover, the protecting groups ($P^2$, $P^3$) for the amino group and carboxy group are also selected from those that can be removed under conditions that hardly cause the elimination of the boryl group and its ester group in Step 4.

The amino-protecting group ($P^2$) is preferably a tert-butoxycarbonyloxy group.

The carboxy-protecting group ($P^3$) is preferably a benzyl group or a $C_{1-2}$ alkyl group (a methyl group, an ethyl group), particularly preferably a benzyl group.

The preferred combinations include

a combination in which the ether-type hydroxy-protecting group ($P^1$) is a benzyl group, the amino-protecting group ($P^2$) is a tert-butoxycarbonyloxy group, and the carboxy-protecting group ($P^3$) is a benzyl group, and

a combination in which the ether-type hydroxy-protecting group ($P^1$) is a p-methoxybenzyl group, the amino-protecting group ($P^2$) is a tert-butoxycarbonyloxy group, and the carboxy-protecting group ($P^3$) is a $C_{1-2}$ alkyl group (a methyl group, an ethyl group).

All of the above protective groups can be removed under mild conditions, and therefore, the elimination of the boryl group or its ester group is difficult to occur.

Each protective group can be introduced according to a method known per se, and the order of the introduction of each protective group is appropriately determined depending on the protective group.

After the completion of the reaction, Compound (3) can be isolated and/or purified from the reaction mixture by separation means such as concentration, crystallization, recrystallization, distillation, solvent extraction, fractional

distillation and chromatography, according to a conventional method.

Step 3 is a step of reacting Compound (3) with a reagent for introducing boronic acid, in the presence of a palladium catalyst and a base to obtain Compound (4).

Examples of the reagent for introducing boronic acid include 4,4,4',4',5,5,5',5'-octamethyl-2,2'-bi-1,3,2-dioxaborolane (also known as bis(pinacolato)diboron), 4,4,5,5-trimethyl-1,3,2-dioxaborolane, tetrahydroxydiborane and the like. Among them, bis(pinacolato)diboron is preferably used. These reagents for introducing boronic acid may be a commercially available product.

The amount of the reagent for introducing boronic acid to be used is generally 1 to 10 mol, preferably 1 to 3 mol, per 1 mol of Compound (3).

Examples of the palladium catalyst include [1,1'-bis(diphenylphosphino)ferrocene]dichloropalladium(II) ($PdCl_2$(dppf)) or its dichloromethane adduct, palladium acetate, tetrakis(triphenylphosphine)palladium(0) ($Pd(PPh_3)_4$) and the like. Among them, $PdCl_2$(dppf) is preferably used.

The amount of the palladium catalyst to be used is generally 0.001 to 1 mol, preferably 0.01 to 0.2 mol, per 1 mol of Compound (3).

Examples of the base include alkali metal acetates such as potassium acetate and sodium acetate, and the like. Among them, alkali metal acetates are preferably used, and potassium acetate is particularly preferably used.

The amount of the base to be used is generally 0.5 to 10 mol, preferably 1 to 5 mol, per 1 mol of Compound (3).

The reaction is carried out generally in a solvent. The solvent to be used in the present invention is not particularly limited as long as it does not adversely affect the reaction, and examples thereof include sulfoxide solvents such as dimethyl sulfoxide; amide solvents such as dimethylformamide, dimethylacetamide and N-methylpyrrolidinone; ether solvents such as tetrahydrofuran and 1,4-dioxane. These may be used in combination of two or more. Among them, sulfoxide solvents and amide solvents are preferably used, and dimethyl sulfoxide is particularly preferably used.

The amount of the solvent to be used is generally 0.1 to 100-fold in volume relative to Compound (3).

The reaction is carried out preferably, for example, by adding (preferably dropwise) a reagent for introducing boronic acid to a mixture of Compound (3), a palladium catalyst, a base and a solvent.

The reaction is carried out generally within the range of 0 to 200°C, preferably room temperature to 120°C. The reaction time varies depending on the reaction temperature, and it is generally about 10 min to about 48 hr, preferably about 1 to about 12 hr.

The completion of the reaction can be confirmed by thin-layer chromatography, liquid chromatography and the like.

After the completion of the reaction, Compound (4) can be isolated and/or purified from the reaction mixture by separation means such as concentration, crystallization, recrystallization, distillation, solvent extraction, fractional distillation, chromatography and the like, according to a conventional method.

Among Compound (4), a compound represented by the following Formula (4a) is a novel compound.

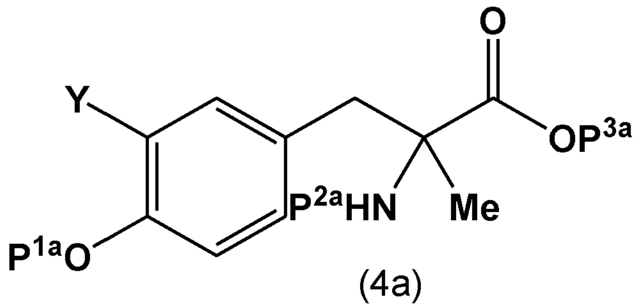

(4a)

wherein

$P^{1a}$ is a benzyl group or a p-methoxybenzyl group;
$P^{2a}$ is a tert-butoxycarbonyl group;
$P^{32}$ is a benzyl group or a $C_{1-2}$ alkyl group; and
Y is as defined above.

Step 4 is a step of removing the protecting groups for the carboxy group, amino group and hydroxy group of Compound (4) to obtain Compound (5).

The removal of each protecting group is carried out according to a method known per se.

For example, when the hydroxy-protecting group ($P^1$)/carboxy-protecting group ($P^3$) are each a benzyl group, the removal is carried out by catalytic hydrogenation.

When the hydroxy-protecting group ($P^1$) is a p-methoxybenzyl group, the removal is carried out by catalytic hydrogenation, or treatment with an acid such as trifluoroacetic acid and hydrogen chloride.

When the carboxy-protecting group ($P^3$) is a $C_{1-2}$ alkyl group (a methyl group, an ethyl group), the removal is carried out by treatment with a base such as lithium hydroxide and sodium hydroxide.

When the amino-protecting group ($P^2$) is a tert-butoxycarbonyl group, the removal is carried out by treatment with an acid such as trifluoroacetic acid and hydrogen chloride.

The above removals of the protecting groups can be carried out under mild conditions, and therefore, the elimination of the boryl group ($-B(OH)_2$) or its ester group is difficult to occur.

After the completion of the reaction, Compound (5) can be isolated and/or purified from the reaction mixture by separation means such as concentration, crystallization, recrystallization, distillation, solvent extraction, fractional distillation, chromatography and the like, according to a conventional method.

Among Compound (5), a compound represented by the following Formula (5a) or a salt thereof is a novel compound.

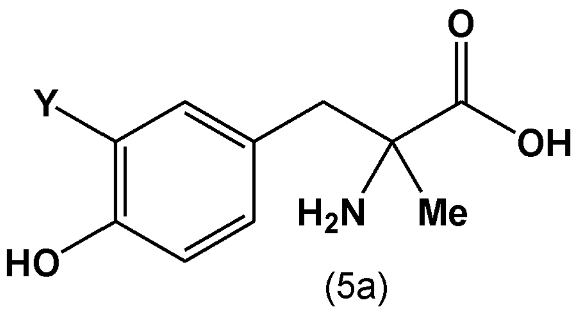

(5a)

wherein Y is as defined above.

Compound (5) thus produced can be converted to Radiolabeled Compound (6) useful as an anti-cancer drug by a method including the following Step 5.

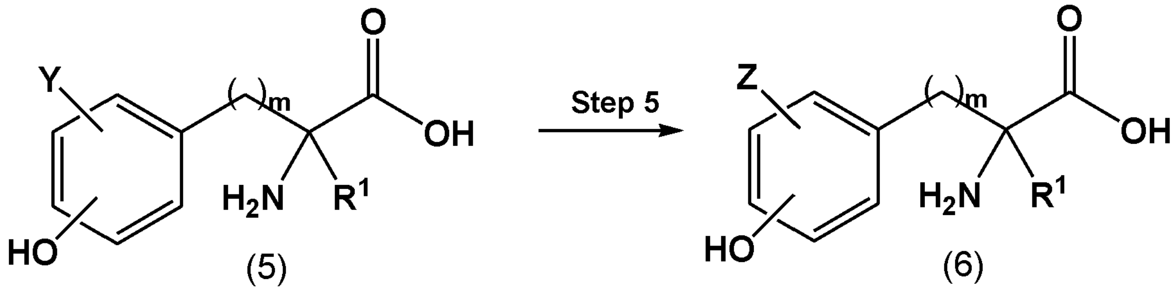


(5) (6)

wherein Z is $^{211}At$, $^{210}At$, $^{123}I$, $^{124}I$, $^{125}I$ or $^{131}I$, and the other symbols are as defined above.

Step 5 is a step of reacting Compound (5) with a radionuclide selected from $^{211}At$, $^{210}At$, $^{123}I$, $^{124}I$, $^{125}I$ and $^{131}I$, in the presence of a reagent selected from an alkali metal iodide, an alkali metal bromide, N-bromosuccinimide, N-chlorosuccinimide, N-iodosuccinimide and hydrogen peroxide, in water to obtain Radiolabeled Compound (6).

Since the reaction in this step is carried out in water, Compound (5) may be in a free form or salt form as long as it can be dissolved in water. Examples of the salt form include a hydrochloride.

Examples of the alkali metal iodide include potassium iodide, sodium iodide and the like. Among them, potassium iodide is preferably used.

Examples of the alkali metal bromide include sodium bromide, potassium bromide and the like.

The preferred combinations of the radionuclide and the above reagent include

(1) a combination in which the radionuclide is $^{211}At$ or $^{210}At$, and the above reagent is selected from potassium iodide, sodium bromide, N-bromosuccinimide, N-chlorosuccinimide, N-iodosuccinimide and hydrogen peroxide; and
(2) a combination in which the radionuclide is $^{123}I$, $^{124}I$, $^{125}I$ or $^{131}I$, and the above reagent is selected from N-bromosuccinimide and N-chlorosuccinimide.

The above reagent may be used alone or in combination of two or more. The above reagent is used usually in the form of an aqueous solution.

As the preferred embodiment, the radionuclide is $^{211}At$ or $^{131}I$, and the reagent is selected from potassium iodide

and N-bromosuccinimide.

The more preferred embodiments include

an embodiment in which the radionuclide is $^{211}At$, and the reagent is potassium iodide, and
an embodiment in which the radionuclide is $^{131}I$, and the reagent is N-bromosuccinimide.

The above reagent is used in an amount sufficient to oxidize or reduce the radionuclide, and is used usually in a large excess amount relative to the radionuclide. It is used preferably in a concentration of 0.0001 to 0.2 mol/L, more preferably in a concentration of 0.001 to 0.1 mol/L, in terms of reaction efficiency and economic efficiency.

For the reaction, the radionuclide is used usually in the form of an aqueous solution. If necessary, an alkaline aqueous solution such as sodium hydroxide and buffer solution may be added to the aqueous solution in order to stabilize the radionuclide.

In the case of radionuclide $^{211}At$, first, $^{211}At$ is produced by $^{209}Bi(\alpha,2n)^{211}At$ nuclear reaction resulting from the irradiation of bismuth with helium particles accelerated to 28 MeV by a cyclotron. Next, by heating, the target substance $^{209}Bi$ is melted and the $^{211}At$ is vaporized, and the vaporized $^{211}At$ is collected in a liquid nitrogen trap, and dissolved in water to prepare an $^{211}At$ stock solution. If necessary, an alkaline solution such as sodium hydroxide and buffer solution may be added thereto for the purpose of stabilizing $^{211}At$.

In the case of radionuclide $^{210}At$, first, $^{210}At$ is produced by $^{209}Bi(\alpha,3n)^{210}At$ nuclear reaction resulting from the irradiation of bismuth with helium particles accelerated to 29 MeV or more by cyclotron. Next, by the same procedures as above, an aqueous $^{210}At$ solution is prepared.

In the case of radionuclide $^{123}I$, it is available as an aqueous $Na^{123}I$ solution.

In the case of radionuclide $^{124}I$, first, $^{124}I$ is produced by $^{124}Te(p,n)^{124}I$ nuclear reaction resulting from the irradiation of tellurium with proton particles accelerated by cyclotron. Next, the target substance $^{124}Te$ is melted, and the $^{124}I$ is vaporized to prepare an aqueous $^{124}I$ sodium hydroxide solution.

In the case of radionuclide $^{125}I$, it is available as an aqueous $Na^{125}I$ solution.

In the case of radionuclide $^{131}I$, it is available as an aqueous $Na^{131}I$ solution.

$^{211}At$ has a half-life of 7.2 hours, $^{210}At$ has a half-life of 8.3 hours, and $^{123}I$ has a half-life of 13.2 hours. These radionuclides have a short half-life, and therefore, they should be used in the subsequent reaction immediately after the preparation. On the other hand, $^{124}I$ has a half-life of 4.2 days, $^{125}I$ has a half-life of 59.4 days, and $^{131}I$ has a half-life of 8.04 days. Although these radionuclides have a relatively long half-life, they are preferably used in the subsequent reaction immediately after the preparation.

Compound (5) is used usually in a large excess amount relative to the radionuclide, preferably in a concentration of 0.0001 mol/l to 0.5 mol/l, more preferably in a concentration of 0.001 mol/l to 0.2 mol/l, per 1 Bq to 1,000 GBq of the radionuclide, in terms of reaction efficiency and economic efficiency.

The above reaction is carried out by mixing Compound (5), the above reagent and the radionuclide, and the mixing order is not particularly limited. The reaction is preferably carried out by adding an aqueous solution of the radionuclide followed by an aqueous solution of the above reagent to an aqueous solution of Compound (5), or by adding an aqueous solution of the above reagent followed by an aqueous solution of the radionuclide to an aqueous solution of Compound (5), more preferably by adding an aqueous solution of the radionuclide followed by an aqueous solution of the above reagent to an aqueous solution of Compound (5).

The above reaction is carried out in water, i.e., in an organic solvent-free system.

The above reaction is carried out at room temperature, specifically within the range of 0°C to 40°C, preferably 10°C to 35°C. In the production method of the present invention, the reaction proceeds rapidly even at room temperature, and is completed in a short time, for example, from 1 minute to 3 hours, especially from 1 min to 30 min.

The completion of the reaction is confirmed by the disappearance of the free radionuclide, using thin-layer chromatography (TLC) analysis.

In the production method of the present invention, Radiolabeled Compound (6) can be obtained in a high radiochemical yield of 60% or more, particularly 80% or more, especially 90% or more.

Since the reaction solution after the completion of the reaction contains neither an organic solvent nor a toxic reagent, the reaction solution can be immediately formulated into an injection and the like without isolating Radiolabeled Compound (6).

Compound (6) may be purified, if necessary, to remove byproducts. This purification is preferably carried out by a solid-phase extraction column. As solid-phase extraction columns, those commonly used in the technical field can be used.

Furthermore, after the above purification, ascorbic acid or ascorbate may be added to a final concentration of 0.01% to 10%, preferably 0.1% to 5%. This makes it possible to suppress the decomposition of Compound (6) and retain it for a long period of time.

The reaction conditions such as solvent and reaction temperature in each step in the production method of the

present invention described above are described in detail as representative examples in the examples below, but are not necessarily limited thereto, and those skilled in the art can make appropriate selections based on their general knowledge in organic synthesis.

## Examples

The present invention will be explained in detail by the following examples, which are merely examples and are not intended to limit the present invention and can be modified without departing from the scope of the present invention which is defined by the appended claims.

In the following Examples, the radiochemical yield was calculated using the following formula.

radiochemical yield (%) = (radioactivity of the desired compound on thin-layer plate/total radioactivity on thin-layer plate) $\times$ 100

### Example 1

Synthesis of 3-(4,4,5,5-tetramethyl-1,3,2-dioxaboran-2-yl)-$\alpha$-methyl-L-tyrosine hydrochloride (5)

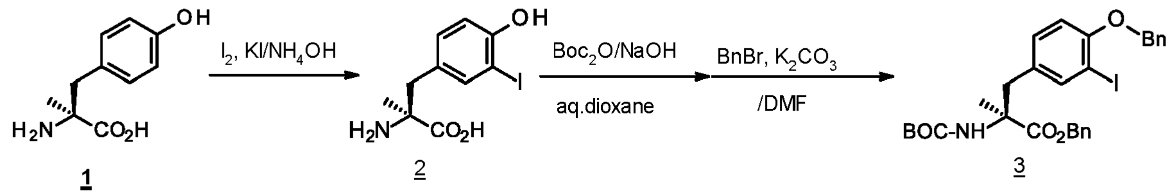


1 2 3

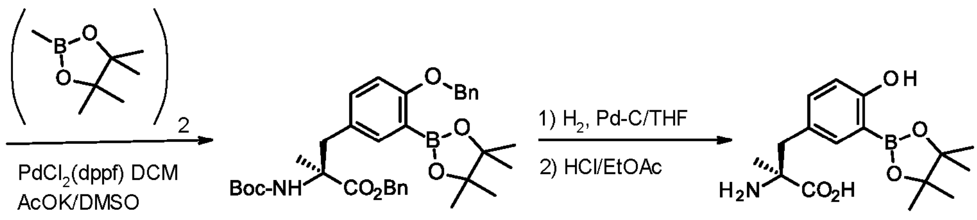


4 5 HCl

a) 3-iodo-$\alpha$-methyl-L-tyrosine (2)

To a mixed solution of potassium iodide (10.2 g, 61.4 mmol) and water (27 mL) was added iodine (13.6 g, 53.6 mmol), and the mixture was stirred for 2 hr. Then, the mixture was added at -5°C or lower to a mixed solution cooled to -7°C of $\alpha$-methyl-L-tyrosine (1) (10 g, 51.2 mmol), conc. aqueous ammonia (28%, 120 mL) and water (15 mL), and the mixture was stirred at -7 to -5°C for 2 hr. To the reaction solution was added 15% aqueous sodium sulfite solution (25 mL), and the mixture was allowed to warm to room temperature, and concentrated under reduced pressure. The pH of the residual solution was adjusted to 6.5 to 7 with 6M hydrochloric acid under ice-cooled, the mixture was stirred under ice-cooled for 1 hr, and the precipitated solid was collected by filtration. The solid was washed with cold water and acetone, and dried under reduced pressure to give 3-iodo-$\alpha$-methyl-L-tyrosine (2) (14.2 g, yield 86%) .

$^1$H-NMR (300MHz, DMSO-$d_6$+TFA, TMS): 8.25(3H, br), 7.51 (1H, d, J=2.1 Hz), 7.03 (1H, dd, J=2.1, 8.1 Hz), 6.84 (1H, d, J=8.1 Hz), 3.03 (1H, d, J=14.1 Hz), 2.87 (1H, d, J=14.1 Hz), 1.44 (3H, s)

b) N-Boc-3-iodo-O-benzyl-$\alpha$-methyl-L-tyrosine benzyl ester (3)

To a mixed solution of 3-iodo-$\alpha$-methyl-L-tyrosine (2) (3.0 g, 9.3 mmol), 1M aqueous sodium hydroxide solution (9.3 mL) and 1,4-dioxane (15 mL) was added di-t-butyl dicarbonate (4.06 g, 18.6 mmol), and the mixture was stirred at 50°C for 24 hr. Additional di-t-butyl dicarbonate (2.0 g, 9.2 mmol) was added thereto, and the mixture was again stirred at 60°C for 20 hr. The reaction solution was allowed to cool to room temperature, and concentrated under reduced pressure.

The residue was subjected to separation extraction with water (30 mL) and methyl t-butyl ether-heptane (1:2, 45 mL). The organic layer was washed with 5% aqueous sodium hydrogencarbonate solution (20 mL) and water (20 mL). The pH of the combined washings was adjusted to 1-2 with 0.5M aqueous sodium hydrogen sulfate solution, and the mixture was subjected to extraction with methyl t-butyl ether-ethyl acetate (1:1, 60 mL). The extract was washed with water and saturated brine. The solvent was evaporated under reduced pressure to give a white solid (2.88 g). The obtained white solid (2.88 g) was dissolved in N,N-dimethylformamide (20 mL), potassium carbonate (2.08 g, 15 mmol) and benzyl bromide (1.8 mL, 15 mmol) were added thereto, and the mixture was stirred at 50°C for 3 hr. The mixture was allowed to cool to room temperature, and ethyl acetate (50 mL) was added thereto. The mixture was washed with water and saturated brine, and the solvent was evaporated under reduced pressure. The residue was purified by silica gel column chromatography to give N-Boc-3-iodo-O-benzyl-α-methyl-L-tyrosine benzyl ester (3) as a white solid (3.39 g, yield 61%). $^1$H-NMR (300MHz, $CDCl_3$, TMS): 7.6-7.3 (11H, m), 6.88 (1H, dd, J=2.1, 8.4 Hz), 6.67 (1H, d, J=8.4 Hz), 5.20 (1H, d, J=12.6 Hz), 5.14 (1H, d, J=12.6 Hz), 5.10 (2H, s), 3.30 (1H, d, J=13.5 Hz), 3.15 (1H, d, J=13.5 Hz), 1.55 (3H, s), 1.48 (9H, s)

c) N-Boc-3-(4,4,5,5-tetramethyl-1,3,2-dioxaboran-2-yl)-O-benzyl-α-methyl-L-tyrosine benzyl ester (4)

Under nitrogen atmosphere, to a mixed solution of N-Boc-3-iodo-O-benzyl-α-methyl-L-tyrosine benzyl ester (3) (3.05 g, 5.0 mmol), [1,1'-bis(diphenylphosphino)ferrocene]dichloropalladium(II) dichloromethane adduct (0.31 g, 0.38 mmol), potassium acetate (1.96 g, 20 mmol) and dimethyl sulfoxide (20 mL) was added bis(pinacolato)diboron (2.54 g, 10 mmol), and the mixture was stirred at room temperature for 1 hr, and then stirred at 55°C for 2 hr. The reaction solution was allowed to cool to room temperature, methyl t-butyl ether (45 mL) and water (45 mL) were added thereto, and the mixture was filtered through Celite. After liquid separation, the aqueous layer was subjected to extraction with methyl t-butyl ether (30 mL), and the organic layers were combined, and washed with water, half-saturated brine and saturated brine, and the solvent was evaporated under reduced pressure. The residue was purified by silica gel column chromatography to give N-Boc-3-(4,4,5,5-tetramethyl-1,3,2-dioxaboran-2-yl)-O-benzyl-α-methyl-L-tyrosine benzyl ester (4) as a white amorphous solid (2.22 g, yield 74%).
$^1$H-NMR (300MHz, $CDCl_3$, TMS): 7.7-7.0 (12H, m), 6.79 (1H, d, J=8.4 Hz), 5.3-5.0 (3H, m), 5.08 (2H, s), 3.31 (1H, d, J=13.8 Hz), 3.18 (1H, d, J=13.8 Hz), 1.46 (9H, s), 1.33 (12H, s), 1.26

(3H, s)

d) 3-(4,4,5,5-tetramethyl-1,3,2-dioxaboran-2-yl)-α-methyl-L-tyrosine hydrochloride (5)

A mixed solution of N-Boc-3-(4,4,5,5-tetramethyl-1,3,2-dioxaboran-2-yl)-O-benzyl-α-methyl-L-tyrosine benzyl ester (4) (2.2 g, 3.6 mmol), 10% palladium-carbon (55% hydrous, 0.55 g) and tetrahydrofuran (22 mL) was stirred under hydrogen gas atmosphere for 3 hr. The reaction solution was filtered through Celite, and the solvent was evaporated under reduced pressure to give a white amorphous solid (1.69 g). To a mixed solution of the obtained amorphous solid (0.56 g, 1.2 mmol) and ethyl acetate (1.5 mL) was added 4M hydrochloric acid-ethyl acetate solution (1.5 mL), and the mixture was stirred at room temperature for 1 hr. The precipitated solid was collected by filtration, and washed with ethyl acetate to give 3-(4,4,5,5-tetramethyl-1,3,2-dioxaboran-2-yl)-α-methyl-L-tyrosine hydrochloride (5) as a white powder (0.30 g, yield 70%). $^1$H-NMR (300MHz, DMSO-$d_6$, TMS): 8.87 (1H, s), 8.35 (3H, br), 7.33 (1H, d, J=2.4 Hz), 7.15 (1H, dd, J=2.4, 8.4 Hz), 6.79 (1H, d, J=8.4 Hz), 3.04 (1H, d, J=14.1 Hz), 2.95 (1H, d, J=14.1 Hz), 1.45 (3H, s), 1.26 (12H, s)
HPLC Analysis Condition
sample solution: 3-(4,4,5,5-Tetramethyl-1,3,2-dioxaboran-2-yl)-α-methyl-L-tyrosine hydrochloride (5) (1 mg) was dissolved in water containing 67% acetonitrile (1 mL) to give a sample solution.
detector: UV 275 nm
column: YMC-Pack Pro C18 RS (4.6 mmφ×25 cm, 5 μm, YMC)
column temperature: 30°C
mobile phase: $H_2O$ (0.1% TFA)/MeCN (0.1% TFA)
mode: gradient

| | | |
|---|---|---|
| 0 min | $H_2O$ | (0.1% TFA)/MeCN (0.1% TFA) = 95/5 |
| 5 min | $H_2O$ | (0.1% TFA)/MeCN (0.1% TFA) = 95/5 |
| 35 min | $H_2O$ | (0.1% TFA)/MeCN (0.1% TFA) = 5/95 |
| 40 min | $H_2O$ | (0.1% TFA)/MeCN (0.1% TFA) = 5/95 |

flow rate: 1 mL/min
injection volume: 5 μL

analysis time: 40 min

retention time: 3-(4,4,5,5-tetramethyl-1,3,2-dioxaboran-2-yl)-α-methyl-L-tyrosine hydrochloride (5): 12.3 min

The HPLC chart is shown in Figure 1.

## Example 2

Synthesis of 3-borono-α-methyl-L-tyrosine hydrochloride (5)

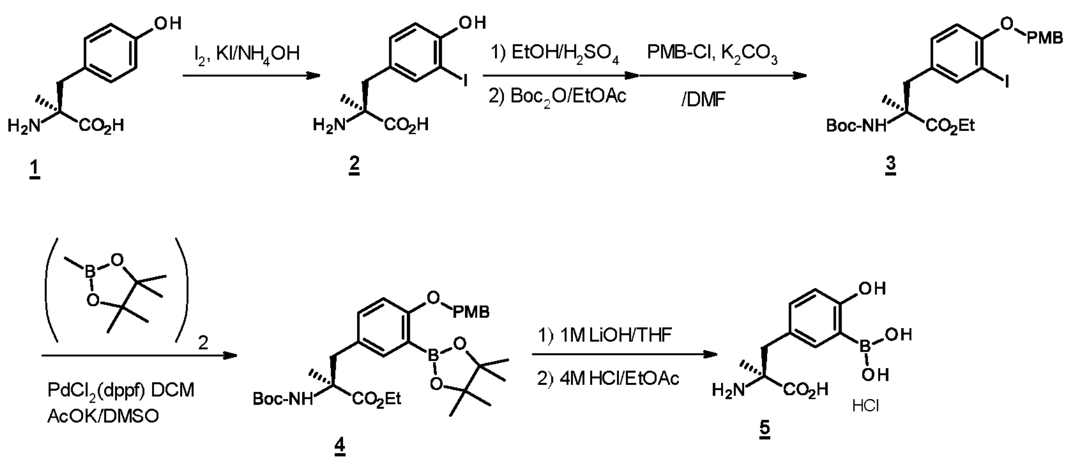


1 2 3 4 5

a) 3-iodo-α-methyl-L-tyrosine (2)

To a mixed solution of potassium iodide (10.2 g, 61.4 mmol) and water (27 mL) was added iodine (13.6 g, 53.6 mmol), and the mixture was stirred for 2 hr. The mixture was added at -5°C or lower to a mixed solution cooled to -7°C of α-methyl-L-tyrosine (1) (10 g, 51.2 mmol), conc. aqueous ammonia (28%, 120 mL) and water (15 mL), and stirred at -7 - -5°C for 2 hr. To the reaction solution was added 15% aqueous sodium sulfite solution (25 mL), and the mixture was allowed to warm to room temperature, and concentrated under reduced pressure. The pH of the residue solution was adjusted to 6.5 to 7 with 6M hydrochloric acid under ice-cooled, and the mixture was stirred under ice-cooled for 1 hr. The precipitated solid was collected by filtration, washed with cold water and acetone, and dried under reduced pressure to give 3-iodo-α-methyl-L-tyrosine (2) (14.2 g, yield 86%).

$^1$H-NMR (300MHz, DMSO-$d_6$+TFA, TMS): 8.25(3H, br), 7.51 (1H, d, J=2.1 Hz), 7.03 (1H, dd, J=2.1, 8.1 Hz), 6.84 (1H, d, J=8.1 Hz), 3.03 (1H, d, J=14.1 Hz), 2.87 (1H, d, J=14.1 Hz), 1.44 (3H, s)

b) N-Boc-3-iodo-O-(p-methoxybenzyl)-α-methyl-L-tyrosine ethyl ester (3)

To a mixed solution of 3-iodo-α-methyl-L-tyrosine (2) (3.0 g, 9.3 mmol) and ethanol (75 mL) was added conc. sulfuric acid (1.0 mL, 18.6 mmol), and the mixture was stirred for 48 hr while heating under reflux. The reaction solution was allowed to cool to room temperature, and concentrated. To the residue were added ice water (30 mL) and sodium hydrogencarbonate (3.9 g), and the mixture was subjected to extraction with ethyl acetate (50 mL). The extract was washed with 5% aqueous sodium hydrogencarbonate solution, water and saturated brine, and the solvent was evaporated under reduced pressure to give a pale-yellow syrup (1.7 g). The obtained residue was dissolved in ethyl acetate (17 mL), and di-t-butyl dicarbonate (1.1 g, 5 mmol) was added thereto under ice-cooled, and the mixture was stirred at room temperature for 2 hr. Additional di-t-butyl dicarbonate (1.1 g, 5 mmol) was added thereto under ice-cooled, and the mixture was stirred at room temperature for 20 hr. The solvent of the reaction solution was evaporated under reduced pressure, and the residue was purified by silica gel column chromatography to give N-Boc-3-iodo-α-methyl-L-tyrosine ethyl ester (Intermediate A) as a white solid (2.0 g). The obtained white solid (1.85 g, 4.1 mmol) was dissolved in N,N-dimethylformamide (10 mL), potassium carbonate (0.68 g, 4.9 mmol) and p-methoxybenzyl chloride (0.6 mL, 4.4 mmol) were added thereto, and the mixture was stirred at 50°C for 2 hr. The mixture was allowed to cool to room temperature, ethyl acetate (50 mL) was added thereto, and the mixture was washed with water and saturated brine. The solvent was evaporated under reduced pressure, and the residue was purified by silica gel column chromatography to give N-Boc-3-iodo-O-(p-methoxybenzyl)-α-methyl-L-tyrosine ethyl ester (3) as a white solid (2.24 g, yield 46%, 3 step). N-Boc-3-iodo-α-methyl-L-tyrosine ethyl ester (Intermediate A) $^1$H-NMR (300MHz, $CDCl_3$, TMS): 7.39 (1H, d, J=2.1 Hz), 6.95 (1H, dd,

J=2.1, 8.4 Hz), 6.87 (1H, d, J=8.4 Hz), 5.28 (1H, br), 5.19(1H, br), 4.23 (2H, q, J=7.2 Hz), 3.33 (1H, d, J=13.5 Hz), 3.12 (1H, d, J=13.5 Hz), 1.61 (3H, s), 1.49 (9H, s), 1.31 (3H, t, J=7.2Hz) N-Boc-3-iodo-O-(p-methoxybenzyl)-α-methyl-L-tyrosine ethyl ester (3)

$^1$H-NMR (300MHz, $CDCl_3$, TMS): 7.52 (1H, d, J=2.4 Hz), 7.40 (2H, d, J=8.7 Hz), 6.98 (1H, dd, J=2.4, 8.4 Hz), 6.90 (2H, d, J=8.7 Hz), 6.75 (1H, d, J=8.4 Hz), 5.18 (1H, br), 5.04 (2H, s), 4.21 (2H, m), 3.82 (3H, s), 3.32 (1H, d, J=13.5 Hz), 3.11 (1H, d, J=13.5 Hz), 1.58 (3H, s), 1.49 (9H, s), 1.31 (3H, t, J=6.9 Hz)

c) N-Boc-3-(4,4,5,5-tetramethyl-1,3,2-dioxaboran-2-yl)-O-(p-methoxybenzyl)-α-methyl-L-tyrosine ethyl ester (4)

Under nitrogen atmosphere, to a mixed solution of N-Boc-3-iodo-O-(p-methoxybenzyl)-α-methyl-L-tyrosine ethyl ester (3) (2.6 g, 4.57 mmol), [1,1'-bis(diphenylphosphino)ferrocene]dichloropalladium(II) dichloromethane adduct (0.22 g, 0.27 mmol), potassium acetate (1.8 g, 18.3 mmol) and dimethyl sulfoxide (20 mL) was added bis(pinacolato) diboron (2.32 g, 9.14 mmol), and the mixture was stirred at room temperature for 1 hr, and then stirred at 55°C for 4 hr. The reaction solution was allowed to cool to room temperature, and methyl t-butyl ether (60 mL) and half-saturated brine (60 mL) were added thereto. The mixture was filtered through Celite, and the filtrate was subjected to liquid separation. The organic layer was washed with half-saturated brine, 5% aqueous sodium hydrogencarbonate solution and saturated brine, and the solvent was evaporated under reduced pressure. The residue was purified by silica gel column chromatography to give N-Boc-3-(4,4,5,5-tetramethyl-1,3,2-dioxaboran-2-yl)-O-(p-methoxybenzyl)-α-methyl-L-tyrosine ethyl ester (4) as a white solid (1.66 g, yield 64%).

$^1$H-NMR (300MHz, $CDCl_3$, TMS): 7.50 (2H, d, J=8.7 Hz), 7.40 (1H, d, J=2.7 Hz), 7.11(1H, dd, J=2.7, 8.4 Hz), 6.89 (2H, d, J=8.7 Hz), 6.83 (1H, d, J=8.4 Hz), 5.18 (1H, br), 5.02 (2H, s), 4.20 (2H, q, J=7.5 Hz), 3.82 (3H, s), 3.32 (1H, d, J=13.2 Hz), 3.13 (1H, d, J=13.2 Hz), 1.60 (3H, s), 1.48 (9H, s), 1.33 (12H, s), 1.29 (3H, t, J=7.5 Hz)

d) 3-borono-α-methyl-L-tyrosine hydrochloride (5)

To a mixed solution of N-Boc-3-(4,4,5,5-tetramethyl-1,3,2-dioxaboran-2-yl)-O-(p-methoxybenzyl)-α-methyl-L-tyrosine ethyl ester (4) (1.3 g, 2.28 mmol), tetrahydrofuran (19.5 mL) and water (6.5 mL) was added lithium hydroxide monohydrate (0.43 g, 10.2 mmol), and the mixture was stirred at room temperature. The reaction solution was concentrated under reduced pressure, and the residue was diluted with water (25 mL). 0.5M Aqueous sodium hydrogen sulfate solution (30 mL) was added thereto (pH 2-3), and the mixture was subjected to extraction with MTBE (30 mL). The extract was washed with water and saturated brine, and the solvent was evaporated under reduced pressure to give a white solid (1.02 g). To the obtained solid was added 4M hydrochloric acid-ethyl acetate solution, the mixture was stirred at room temperature for 30 min, and the solvent was evaporated under reduced pressure. The residue was washed with ethyl acetate, and dried to give crude 3-borono-α-methyl-L-tyrosine hydrochloride (5) (0.42 g) as a white powder. This powder (0.40 g) was dissolved in a small amount of $H_2O$, and subjected to preparative purification with ODS separation column under conditions of mobile phase: 0.1% HCl aq./acetonitrile and gradient: 100/0 to 95/5. The objective fractions were collected, and the solvent was evaporated. The residue was dissolved in 0.1% HCl aq., and subjected to cotton-plug filtration. The solvent was evaporated under reduced pressure, and the residue was dried under reduced pressure by vacuum pump to give 3-borono-α-methyl-L-tyrosine hydrochloride (5) (283 mg, yield 45%).

$^1$H-NMR (300MHz, DMSO-$d_6$+5% D2O, TMS): 8.42 (1H, s), 7.46 (1H, m, J=2.4 Hz), 7. 00-7.17 (1H, m), 6.72-6.82 (1H, m), 3.06 (1H, d, J=14.1 Hz), 2.95 (1H, d, J=14.1 Hz), 1.47 (3H, s)

HPLC Analysis Condition

sample solution: 3-Borono-α-methyl-L-tyrosine hydrochloride (5) (1 mg) was dissolved in 0.1% HCl aq. (1 mL) to give a sample solution.

detector: UV 275 nm

column: YMC-Pack Pro C18 RS (4.6 mmφ×25 cm, 5 μm, YMC)

column temperature: 30°C

mobile phase: $H_2O$ (0.1% TFA)/MeCN (0.1% TFA)

mode: gradient

| | | |
|---|---|---|
| 0 min | $H_2O$ | (0.1% TFA)/MeCN (0.1% TFA) = 95/5 |
| 5 min | $H_2O$ | (0.1% TFA)/MeCN (0.1% TFA) = 95/5 |
| 35 min | $H_2O$ | (0.1% TFA)/MeCN (0.1% TFA) = 5/95 |
| 40 min | $H_2O$ | (0.1% TFA)/MeCN (0.1% TFA) = 5/95 |

flow rate: 1 mL/min

injection volume: 5 μL

analysis time: 40 min
retention time: 3-borono-α-methyl-L-tyrosine hydrochloride (5): 11.7 min, 12.2 min (detected as a mixture of the boroxine (associated molecule) and the monomer)

The HPLC chart is shown in Figure 2.

Reference Example 1

Preparation of $^{211}At$ aqueous solution

According to the method described in Reference Example 1 of Patent Document 2, $^{211}At$ aqueous solution was prepared. That is, $^{211}At$ was produced by $^{209}Bi$ (α, 2n) $^{211}At$ nuclear reaction resulting from the irradiation of bismuth with helium particles accelerated (28 MeV) by a cyclotron. After the irradiation, by heating, the target substance $^{209}Bi$ was melted, while the $^{211}At$ was vaporized. The vaporized $^{211}At$ was collected to a cooled trap, and dissolved in a small amount of water to prepare an $^{211}At$ aqueous solution.

Example 3

Synthesis of 3-astato($^{211}At$)-α-methyl-L-tyrosine

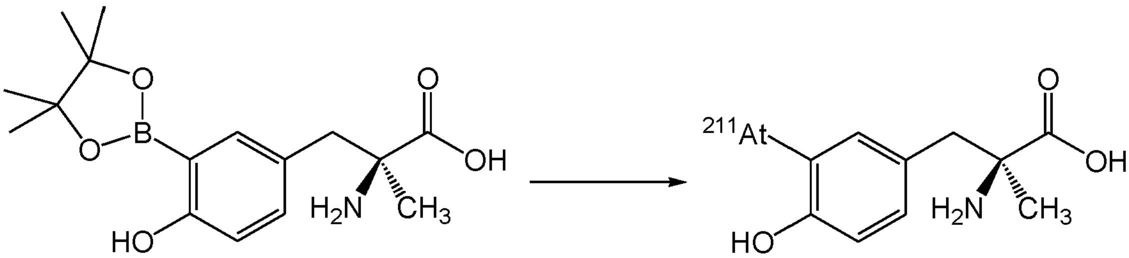

Bpin-AMT $^{211}At$-AAMT

3-(4,4,5,5-Tetramethyl-1,3,2-dioxaboran-2-yl)-α-methyl-L-tyrosine hydrochloride (Bpin-AMT) was dissolved in water to prepare a 0.8 w/v% aqueous solution. This aqueous solution (0.1 mL) was put in a microtube, and then the $^{211}At$ aqueous solution (0.02 mL, 5 MBq) prepared in Reference Example 1 and 0.1M aqueous KI solution (0.05 mL) were added thereto, and the mixture was reacted at 50°C for 1 hr to give a crude product solution of 3-astato ($^{211}At$) -α-methyl-L-tyrosine ($^{211}At$-AAMT).

This crude product solution (1 μL) was analyzed by thin-layer chromatograph method (TLC). The sample was spotted on a thin-layer plate (silica gel G60), and a mixed solvent of acetonitrile/water (2/1) was used as a developing solvent. The thin-layer plate was exposed to an imaging plate for about 15 min, and then analyzed by a bioimage analyzer (Typhoon FLT7000, GE Healthcare, Chicago). The results of the TLC analysis are shown in Figure 3. 3-Astato($^{211}At$)-α-methyl-L-tyrosine in the crude product solution was detected at Rf 0.78, and the radiochemical yield (RCY) was 74.9%.

The crude product solution was injected into an Oasis HLB cartridge (Waters) to capture the target product, and water (1 mL) was passed through the cartridge to elute impurities. Next, 30% ethanol solution (1 mL) was passed through the cartridge to elute the target product to give the purified product. The radiochemical yield and radiochemical purity of the purified product were 74% and 94.8%, respectively.

Example 4

Synthesis of 3-astato($^{211}At$)-α-methyl-L-tyrosine

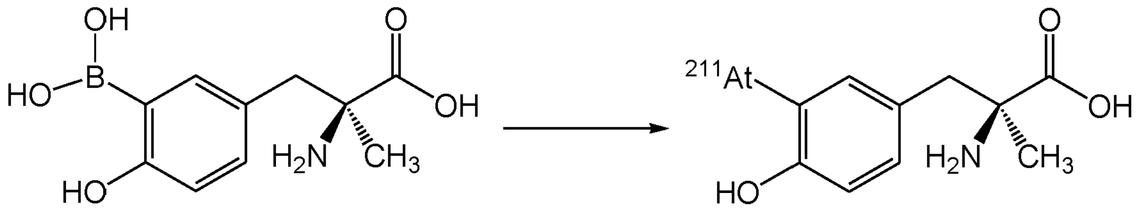

Borono-AMT

$^{211}$At-AAMT

3-Borono-α-methyl-L-tyrosine hydrochloride (Borono-AMT) was dissolved in water to prepare a 1 w/v% aqueous solution. This aqueous solution (0.1 mL) was put in a microtube, and then the $^{211}$At aqueous solution (0.02 mL, 5 MBq) prepared in Reference Example 1 and 0.1M aqueous KI solution (0.02 mL) were added thereto, and the mixture was reacted at 50°C for 1 hr to give a crude product solution of 3-astato($^{211}$At)-α-methyl-L-tyrosine ($^{211}$At-AAMT).

This crude product solution (1 μL) was analyzed by thin-layer chromatograph method (TLC). The sample was spotted on a thin-layer plate (silica gel G60), and a mixed solvent of acetonitrile/water (2/1) was used as a developing solvent. The thin-layer plate was exposed to an imaging plate for about 15 min, and then analyzed by a bioimage analyzer (Typhoon FLT7000, GE Healthcare, Chicago). The results of the TLC analysis are shown in Figure 4. 3-Astato($^{211}$At)-α-methyl-L-tyrosine was detected at Rf 0.78, and the radiochemical yield (RCY) was 68.2%.

The crude product solution was injected into an Oasis HLB cartridge to capture the target product, and water (1 mL) was passed through the cartridge to elute impurities. Next, 30% ethanol solution (1 mL) was passed through the cartridge to elute the target product to give the purified product. The radiochemical yield and radiochemical purity of the purified product were 58.3% and 90.9%, respectively.

Example 5

Synthesis of 3-iodo($^{131}$I)-α-methyl-L-tyrosine

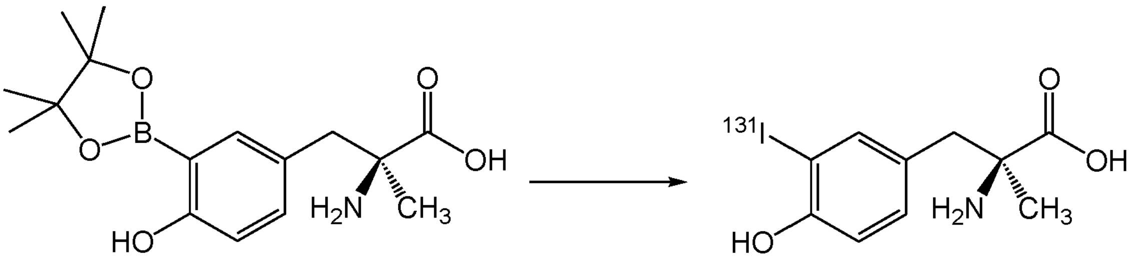

Bpin-AMT

$^{131}$I-IAMT

3-(4,4,5,5-Tetramethyl-1,3,2-dioxaboran-2-yl)-α-methyl-L-tyrosine hydrochloride (Bpin-AMT) was dissolved in water to prepare a 1 w/v% aqueous solution. This aqueous solution (0.1 mL) was put in a microtube, and then aqueous [$^{131}$I]sodium iodide solution (0.03 mL, 1 MBq, Institute of Isotopes Co., Ltd.) and 0.4 w/v% aqueous N-bromosuccinimide (NBS) solution (0.03 mL) were added thereto, and the mixture was reacted at room temperature for 30 min. Then, to the reaction solution was added 3 w/v% aqueous ascorbic acid solution (0.03 mL) to stop the reaction to give a crude product solution of 3-iodo($^{131}$I)-α-methyl-L-tyrosine ($^{131}$I-IAMT).

This crude product solution (1 μL) was analyzed by thin-layer chromatograph method (TLC). The sample was spotted on a thin-layer plate (silica gel G60), and a mixed solvent of acetonitrile/water (2/1) was used as a developing solvent. The thin-layer plate was exposed to an imaging plate for about 15 min, and then analyzed by a bioimage analyzer (Typhoon FLT7000, GE Healthcare, Chicago). The results of the TLC analysis are shown in Figure 5. 3-Iodo($^{131}$I)-α-methyl-L-tyrosine was detected at Rf 0.72, and the radiochemical yield (RCY) was 86.1%. This compound was stable up to 7 days after labeling.

Reference Example 2

Synthesis of 3-astato($^{211}$At)-α-methyl-L-tyrosine

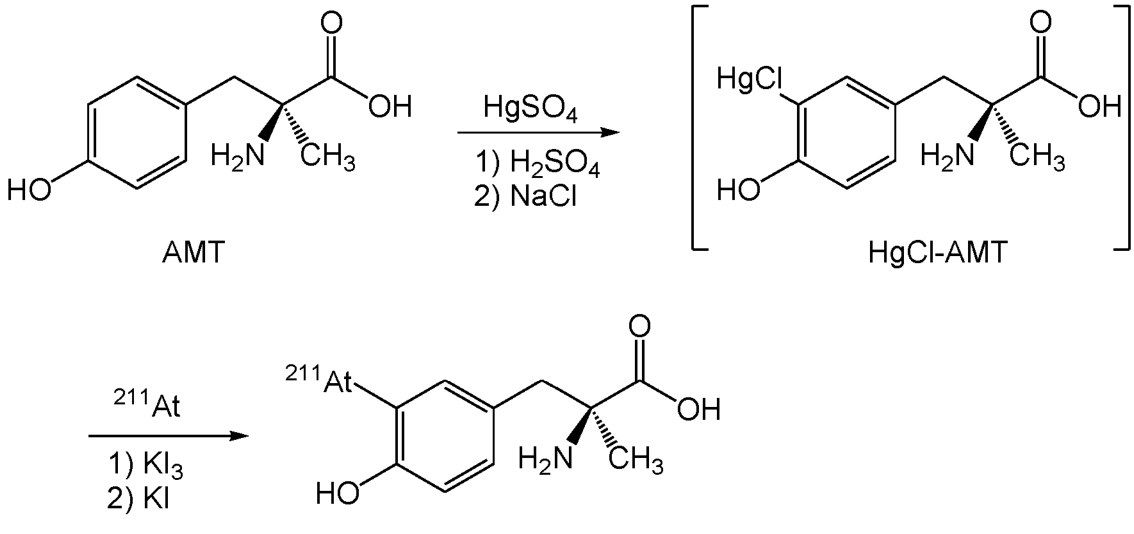


AMT

HgCl-AMT

$^{211}$At-AAMT

α-Methyl-L-tyrosine (AMT) (22 μmol) and HgSO$_4$ (20 μmol) were added to $H_2SO_4$ aqueous solution (0.2 M, 0.5 mL), and the mixture was stirred at room temperature for 2 hr. NaCl (45 μmoL) was added to the reaction solution, and the mixture was stirred for 5 min. To the reaction solution were added the $^{211}$At aqueous solution (about 5 MBq/mL, 100 μL) prepared in Reference Example 1 and 1M KI$_3$ (5 μL), and the mixture was stirred for 30 min. Then, an appropriate amount (100 μL or more) of 1M KI solution was added thereto until the suspension became clear to stop the reaction. The obtained reaction solution was desalted through a cation column (Dowex TM 50Wx8 100-200 mesh, 0.2M aqueous NH$_3$ solution), followed by an anion column (Dowex TM 1x8 50-100 mesh, 2% aqueous AcOH solution) to give 3-astato($^{211}$At)-α-methyl-L-tyrosine ($^{211}$At-AAMT). Furthermore, sodium ascorbate was added thereto to a final concentration of 1 w/v% for stabilization. The yield was 60 to 80%.

Example 6

This example demonstrates that the $^{211}$At-AAMT synthesized by the method of the present invention is taken up by cancer cells via an amino acid transporter (LAT1) that is specifically expressed in cancer cells, and that this performance is comparable to that of the $^{211}$At-AAMT synthesized by the conventional mercury method.

Human pancreatic cancer cells MIA PaCa-2 were seeded in a 24-well culture plate at a concentration of $1x10^5$ cells/mL. After 2 days, the medium was removed, the cells were washed with PBS (-), and the medium was replaced with HEPES Buffer (amino acid free). To each well, the $^{211}$At-AAMT synthesized by the method of the present invention or the conventional mercury method was added. In addition, an excess amount (1 mM) of BCH (LAT1 inhibitor) or unlabeled (AMT) was added. After culturing the cells for 30 minutes, the cells were washed with PBS (-), and lysed with 0.1 N NaOH, and the radioactivity of the lysate was measured. The $^{211}$At-AAMT intracellular uptake (radioactivity count/protein amount) was calculated by correcting the radioactivity per protein amount. The results are shown in Figure 6 (Figure 6a: the method of the present invention, Figure 6b: the conventional mercury method). In Figure 6, CTL is control, BCH is 2-aminobicyclo [2,2,1]heptane-2-carboxylic acid, and AMT is α-methyltyrosine. The $^{211}$At-AAMT synthesized by the method of the present invention was strongly taken up by pancreatic cancer cells (CTL), and the uptake was significantly inhibited in the presence of BCH and AMT. This result was consistent with that of the $^{211}$At-AAMT synthesized by conventional techniques. From these results, it was confirmed that the $^{211}$At-AAMT synthesized by the method of the present invention is specifically taken up into pancreatic cancer cells via LAT1.

Example 7

This example demonstrates that the $^{211}$At-AAMT synthesized by the method of the present invention has cytotoxicity against cancer cells, and that this performance is comparable to that of the $^{211}$At-AAMT synthesized by the conventional mercury method.

HEK293 cells were seeded in a 24-well culture plate at a concentration of $1x10^5$ cells/mL. After 2 days, the

medium was removed, the cells were washed with PBS (-), and the medium was replaced with HEPES Buffer (amino acid free). To each well, 0.00-5.00 kBq of the $^{211}$At-AAMT synthesized by the method of the present invention or the conventional mercury method was added. After culturing the cells for 24 hours, the cell viability was examined. The results are shown in Figure 7. In the group of the $^{211}$At-AAMT synthesized by the method of the present invention, the cell viability decreased in a dose-dependent manner. This result was consistent with that of the group of the $^{211}$At-AAMT synthesized by the conventional mercury method. From these results, it was confirmed that the $^{211}$At-AAMT synthesized by the method of the present invention has the same cytotoxicity as that of the conventional mercury method.

**Industrial Applicability**

According to the present invention, tyrosine derivatives into which a boryl group ($-B(OH)_2$) or its ester group is introduced can be obtained, and therefore, the use of the compounds makes it possible to produce radiolabeled tyrosine derivatives with good purity and stability, by a safe method suitable for industrial production of pharmaceuticals without using hazardous substances.

**Claims**

1. A method for producing a compound represented by Formula (5) or a salt thereof, comprising the following Steps 1 to 4;

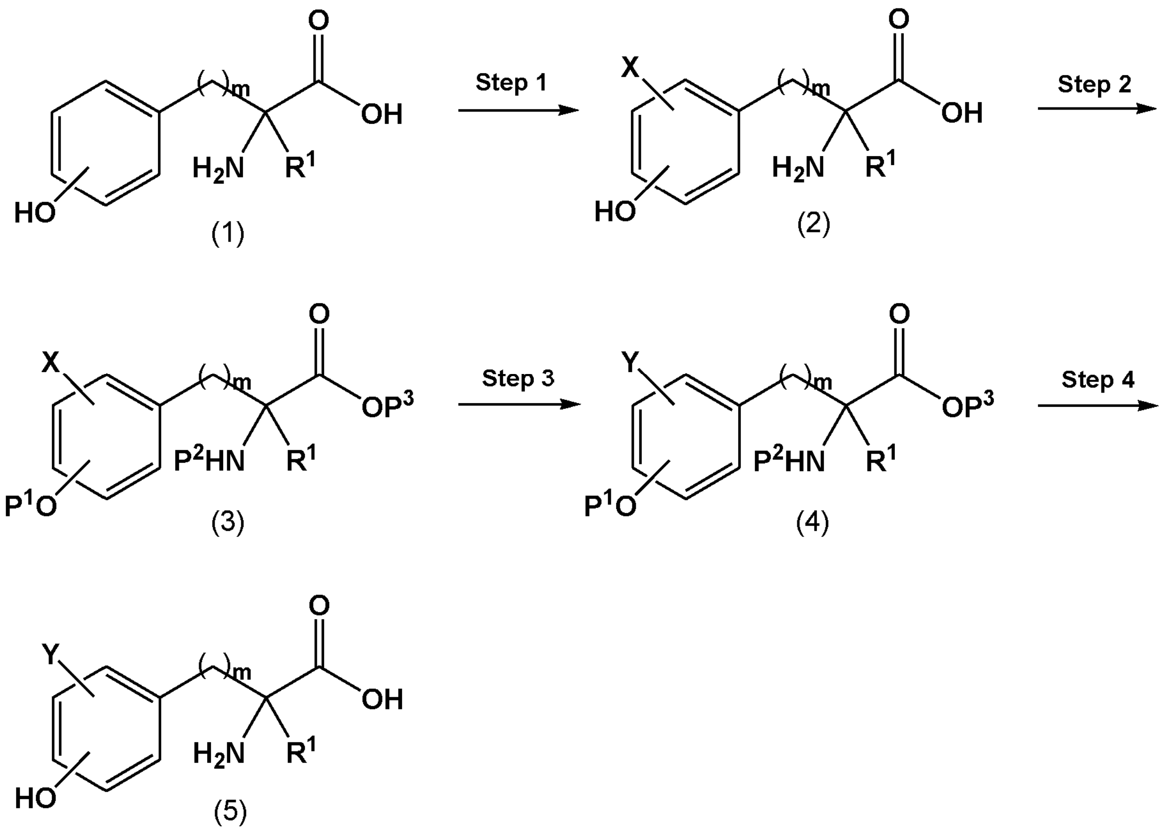

(1) (2) (3) (4) (5)

wherein

$R^1$ is a methyl group;
$P^1$ is an ether-type hydroxy-protecting group;
$P^2$ is an amino-protecting group;
$P^3$ is a carboxy-protecting group;
m is 0, 1 or 2;
X is a halogen atom; and
Y is a boryl group ($-B(OH)_2$) or its ester group,

Step 1: a step of halogenating a compound represented by Formula (1) or a salt thereof to obtain a compound represented by Formula (2) or a salt thereof;
Step 2: a step of protecting the amino group and carboxy group of the compound represented by Formula (2) or a salt thereof and protecting the hydroxy group of the compound or salt with an ether-type protecting group to obtain a compound represented by Formula (3);
Step 3: a step of reacting the compound represented by Formula (3) with a reagent for introducing boronic acid in the presence of a palladium catalyst and a base to obtain a compound represented by Formula (4); and
Step 4: a step of removing the protecting groups for the carboxy group, amino group and hydroxy group of the compound represented by Formula (4) to obtain the compound represented by Formula (5) or a salt thereof.

**2.** The method according to Claim 1, wherein $P^1$ is a benzyl group or a p-methoxybenzyl group.

**3.** The method according to Claim 1 or 2, wherein $P^3$ is a benzyl group or a $C_{1-2}$ alkyl group.

**4.** The method according to Claim 1, wherein $P^1$ and $P^3$ are both benzyl groups.

**5.** The method according to any one of Claims 1 to 4, wherein the bonding position of the hydroxy group on the benzene ring in Formula (5) is the 4-position or 3-position, or wherein the bonding positions of the hydroxy group and the group -Y on the benzene ring in Formula (5) are preferably adjacent to each other.

**6.** The method according to any one of Claims 1 to 4, wherein, in Formula (5), the bonding position of the hydroxy group on the benzene ring is the 4-position, and the bonding position of the group -Y on the benzene ring is the 3-position.

**7.** The method according to any one of Claims 1 to 6, wherein the palladium catalyst to be used in Step 3 is [1,1'-bis(diphenylphosphino)ferrocene]dichloropalladium(II) ($PdCl_2$(dppf)), wherein the reaction of Step 3 is preferably carried out in a sulfoxide solvent or an amide solvent, or wherein the base to be used in Step 3 is preferably an alkali metal acetate.

**8.** The method according to any one of Claims 1 to 7, wherein Y is a boryl group ($-B(OH)_2$) or a 4,4,5,5-tetramethyl-1,3,2-dioxaboran-2-yl group.

**9.** A compound represented by the following Formula (5a) or a salt thereof;

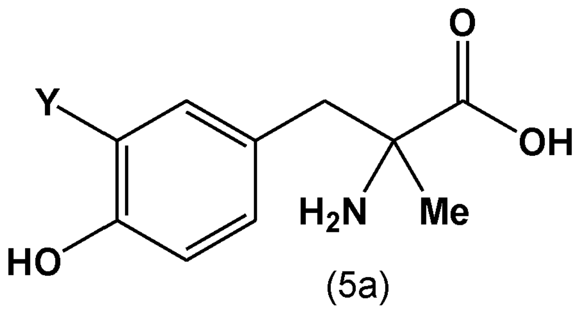

(5a)

wherein Y is a boryl group ($-B(OH)_2$) or its ester group.

**10.** A compound represented by the following Formula (4a);

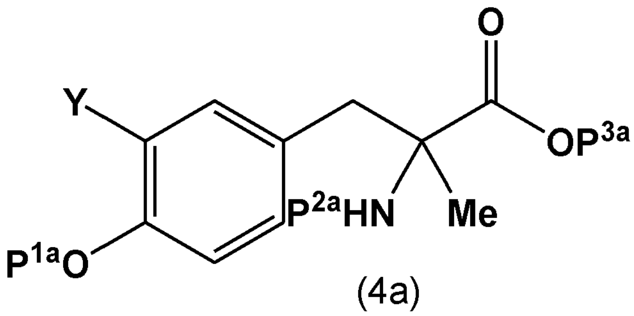

(4a)

wherein

P^1a^ is a benzyl group or a p-methoxybenzyl group;
$P^{2a}$ is a tert-butoxycarbonyl group;
$P^{32}$ is a benzyl group or a $C_{1-2}$ alkyl group; and
Y is a boryl group ($-B(OH)_2$) or its ester group.

**11.** A method for producing a radiolabeled compound represented by Formula (6) or a salt thereof, comprising the following Step 5;

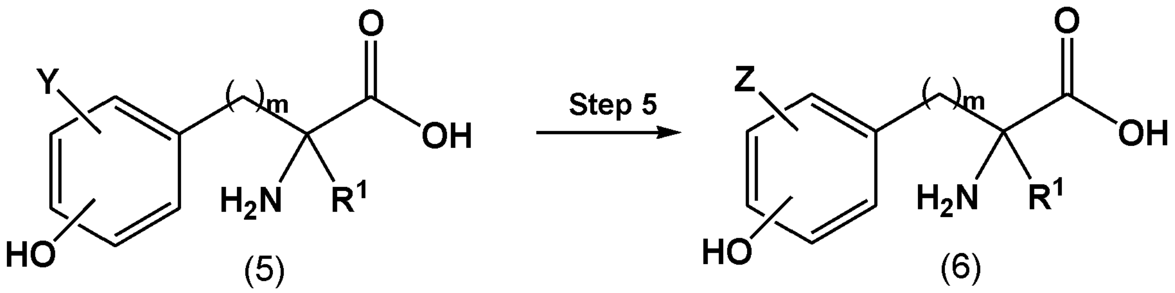


(5) (6)

wherein

$R^1$ is a hydrogen atom or a $C_{1-4}$ alkyl group;
m is 0, 1 or 2;
Y is a boryl group ($-B(OH)_2$) or its ester group; and
Z is $^{211}At$, $^{210}At$, $^{123}I$, $^{124}I$, $^{125}I$ or $^{131}I$,
Step 5: a step of reacting a compound represented by Formula (5) or a salt thereof with a radionuclide selected from $^{211}At$, $^{210}At$, $^{123}I$, $^{124}I$, $^{125}I$ and $^{131}I$, in the presence of a reagent selected from an alkali metal iodide, an alkali metal bromide, N-bromosuccinimide, N-chlorosuccinimide, N-iodosuccinimide and hydrogen peroxide, in water to obtain the radiolabeled compound represented by Formula (6) or a salt thereof.

**12.** The method according to Claim 11, wherein the compound represented by Formula (5) or a salt thereof is produced by a method as defined in any one of Claims 1 to 12 and/or wherein the reaction is carried out in an organic solvent-free system, and/or wherein the reaction is carried out within the range of room temperature to 100°C, and/or wherein the radionuclide is $^{211}At$ or $^{131}I$, and the reagent is selected from potassium iodide and N-bromosuccinimide, and/or further comprising a step of purifying the radiolabeled compound represented by Formula (6) or a salt thereof, and/or further comprising a step of stabilizing the radiolabeled compound represented by Formula (6) or a salt thereof by adding ascorbic acid.

**Patentansprüche**

**1.** Verfahren zur Herstellung einer Verbindung der Formel (5) oder eines Salzes davon, umfassend die folgenden Schritte 1 bis 4;

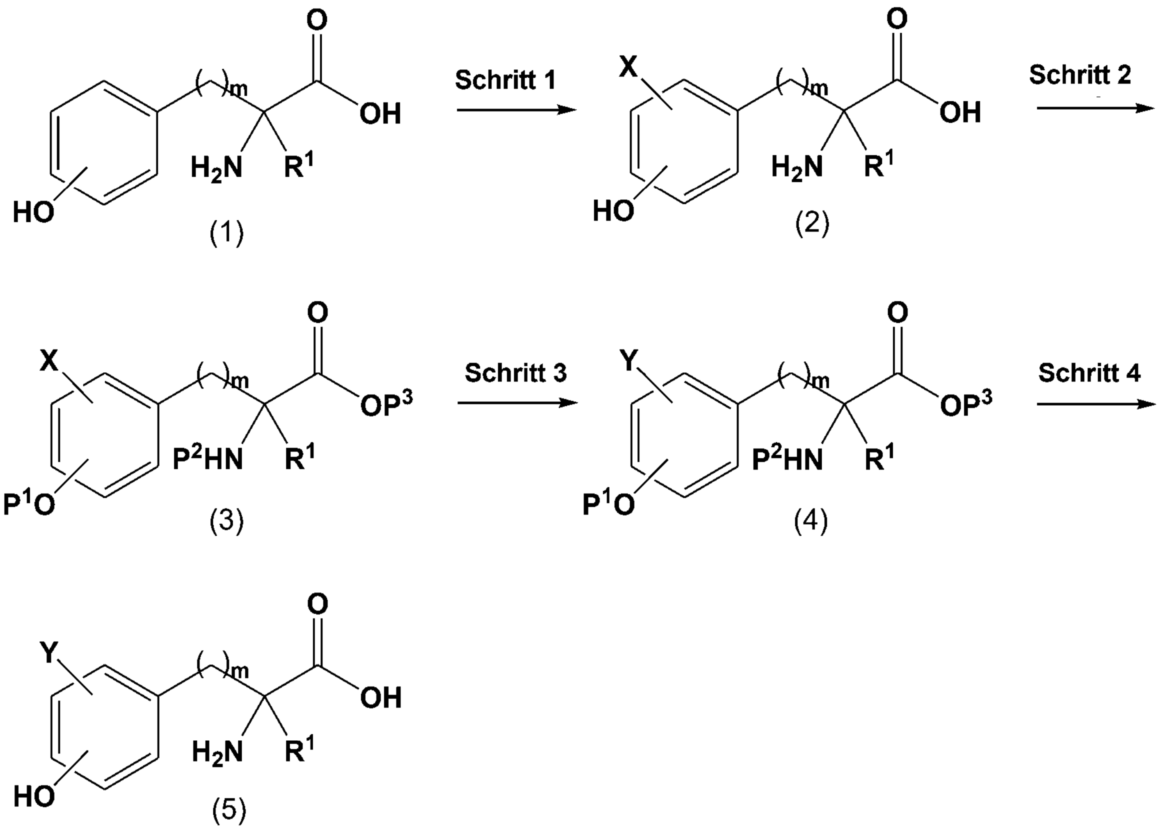


(1) (2) (3) (4) (5)

worin

$R^1$ eine Methylgruppe ist;
$P^1$ eine Hydroxy-Schutzgruppe vom Ethertyp ist;
$P^2$ eine Amino-Schutzgruppe ist;
$P^3$ eine Carboxy-Schutzgruppe ist;
m 0, 1 oder 2 ist;
X ein Halogenatom ist; und
Y eine Borylgruppe ($-B(OH)_2$) oder deren Estergruppe ist,
Schritt 1: ein Schritt der Halogenierung einer Verbindung der Formel (1) oder eines Salzes davon, um eine Verbindung der Formel (2) oder ein Salz davon zu erhalten;
Schritt 2: ein Schritt des Schützens der Aminogruppe und der Carboxygruppe der Verbindung der Formel (2) oder eines Salzes davon sowie der Hydroxygruppe der Verbindung oder des Salzes mit einer Schutzgruppe vom Ethertyp, um eine Verbindung der Formel (3) zu erhalten;
Schritt 3: ein Schritt des Umsetzens der Verbindung der Formel (3) mit einem Reagenz zur Einführung von Boronsäure in Gegenwart eines Palladiumkatalysators und einer Base, um eine Verbindung der Formel (4) zu erhalten; und
Schritt 4: ein Schritt zum Entfernen der Schutzgruppen für die Carboxygruppe, die Aminogruppe und die Hydroxygruppe der Verbindung der Formel (4), um die Verbindung der Formel (5) oder ein Salz davon zu erhalten.

**2.** Verfahren nach Anspruch 1, wobei $P^1$ eine Benzylgruppe oder eine p-Methoxybenzylgruppe ist.

**3.** Verfahren nach Anspruch 1 oder 2, wobei $P^3$ eine Benzylgruppe oder eine $C_{1-2}$-Alkylgruppe ist.

**4.** Verfahren nach Anspruch 1, wobei $P^1$ und $P^3$ beide Benzylgruppen sind.

**5.** Verfahren nach einem der Ansprüche 1 bis 4, wobei die Bindungsposition der Hydroxygruppe am Benzolring in Formel (5) die 4-Position oder die 3-Position ist, oder wobei die Bindungspositionen der Hydroxygruppe und der Gruppe -Y am Benzolring in Formel (5) vorzugsweise benachbart zueinander sind

6. Verfahren nach einem der Ansprüche 1 bis 4, wobei in Formel (5) die Bindungsposition der Hydroxygruppe am Benzolring die 4-Position ist und die Bindungsposition der Gruppe -Y am Benzolring die 3-Position ist.

7. Verfahren nach einem der Ansprüche 1 bis 6, wobei der in Schritt 3 zu verwendende Palladiumkatalysator [1,1'-Bis(diphenylphosphino)ferrocen]dichlorpalladium (II) ($PdCl_2$(dppf)) ist, wobei die Reaktion von Schritt 3 vorzugsweise in einem Sulfoxid-Lösungsmittel oder einem Amid-Lösungsmittel durchgeführt wird, oder wobei die in Schritt 3 zu verwendende Base vorzugsweise ein Alkalimetallacetat ist.

8. Verfahren nach einem der Ansprüche 1 bis 7, wobei Y eine Borylgruppe ($-B(OH)_2$) oder eine 4,4,5,5-Tetramethyl-1,3,2-dioxaboran-2-yl-Gruppe ist.

9. Verbindung der folgenden Formel (5a) oder ein Salz davon;

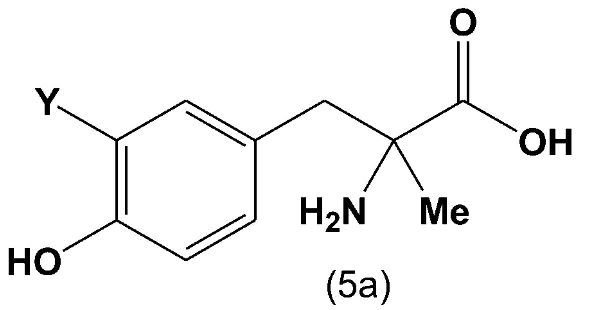

(5a)

wobei Y eine Borylgruppe ($-B(OH)_2$) oder deren Estergruppe ist.

10. Verbindung der folgenden Formel (4a);

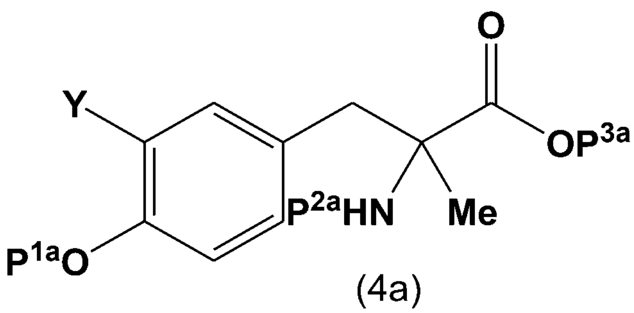

(4a)

wobei

$P^{1a}$ eine Benzylgruppe oder eine p-Methoxybenzylgruppe ist;
$P^{2a}$ eine tert-Butoxycarbonylgruppe ist;
$P^{3a}$ eine Benzylgruppe oder eine $C_{1-2}$-Alkylgruppe ist; und
Y eine Borylgruppe ($-B(OH)_2$) oder deren Estergruppe ist.

11. Verfahren zur Herstellung einer radioaktiv markierten Verbindung der Formel (6) oder eines Salzes davon, umfassend den folgenden Schritt 5;

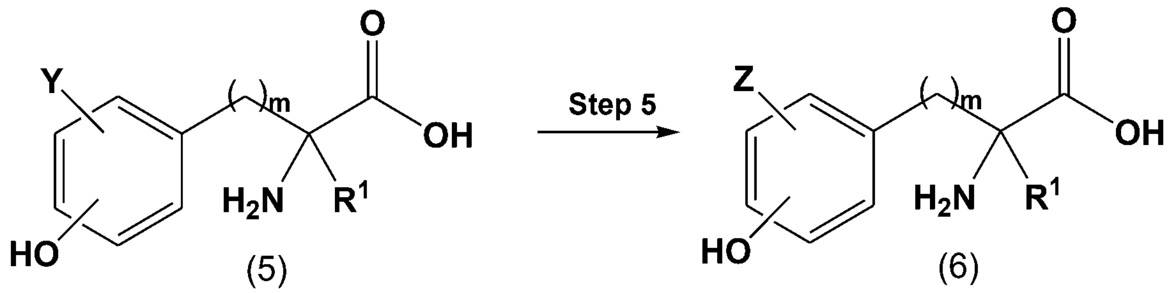


wobei

$R^1$ ein Wasserstoffatom oder eine $C_{1-4}$-Alkylgruppe ist;
m 0, 1 oder 2 ist;
Y eine Borylgruppe ($-B(OH)_2$) oder deren Estergruppe ist; und
Z $^{211}At$, $^{210}At$, $^{123}I$, $^{124}I$, $^{125}I$ oder $^{131}I$ ist,
Schritt 5: ein Schritt der Umsetzung einer Verbindung der Formel (5) oder eines Salzes davon mit einem Radionuklid, ausgewählt aus $^{211}At$, $^{210}At$, $^{123}I$, $^{124}I$, $^{125}I$ und $^{131}I$, in Gegenwart eines Reagenzes, ausgewählt aus einem Alkalimetalliodid, einem Alkalimetallbromid, N-Bromsuccinimid, N-Chlorsuccinimid, N-Iodosuccinimid und Wasserstoffperoxid, in Wasser, um die durch die Formel (6) dargestellte radioaktiv markierte Verbindung oder ein Salz davon zu erhalten.

**12.** Verfahren nach Anspruch 11, wobei die Verbindung der Formel (5) oder ein Salz davon durch ein Verfahren gemäß einem der Ansprüche 1 bis 12 hergestellt wird und/oder wobei die Reaktion in einem organischen lösungsmittelfreien System durchgeführt wird und/oder wobei die Reaktion im Bereich von Raumtemperatur bis 100 °C durchgeführt wird, und/oder wobei das Radionuklid $^{211}At$ oder $^{131}I$ ist und das Reagenz aus Kaliumiodid und N-Bromsuccinimid ausgewählt ist, und/oder ferner umfassend einen Schritt zum Reinigen der durch die Formel (6) dargestellten radioaktiv markierten Verbindung oder eines Salzes davon, und/oder ferner umfassend einen Schritt zum Stabilisieren der durch die Formel (6) dargestellten radioaktiv markierten Verbindung oder eines Salzes davon durch Zugabe von Ascorbinsäure.

**Revendications**

**1.** Procédé pour produire un composé représenté par la formule (5) ou un sel de celui-ci, comprenant les étapes 1 à 4 suivantes ;

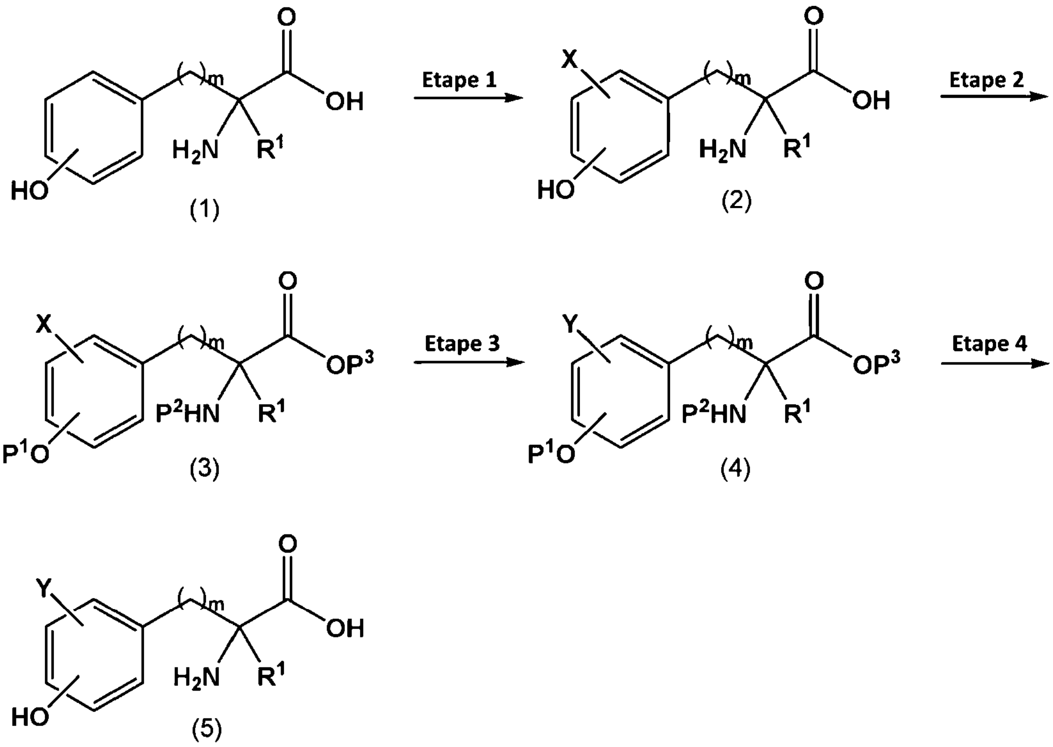


(1) (2) (3) (4) (5)

dans lequel

$R^1$ est un groupe méthyle ;
$P^1$ est un groupe hydroxy-protecteur de type éther ;
$P^2$ est un groupe amino-protecteur ;
$P^3$ est un groupe carboxy-protecteur ;
m vaut 0, 1 ou 2 ;
X est un atome d'halogène ; et
Y est un groupe boryle ($-B(OH)_2$) ou son groupe ester,
étape 1 : une étape d'halogénation d'un composé représenté par la formule (1) ou d'un sel de celui-ci pour que soit obtenu un composé représenté par la formule (2) ou un sel de celui-ci ;
étape 2 : une étape de protection du groupe amino et du groupe carboxy du composé représenté par la formule (2) ou d'un sel de celui-ci et de protection du groupe hydroxy du composé ou sel avec un groupe protecteur de type éther pour que soit obtenu un composé représenté par la formule (3) ;
étape 3 : une étape de réaction du composé représenté par la formule (3) avec un réactif pour introduire de l'acide boronique en présence d'un catalyseur au palladium et d'une base pour que soit obtenu un composé représenté par la formule (4) ; et
étape 4 : une étape d'élimination des groupes protecteurs pour le groupe carboxy, le groupe amino et le groupe hydroxy du composé représenté par la formule (4) pour que soit obtenu le composé représenté par la formule (5) ou un sel de celui-ci.

**2.** Procédé selon la revendication 1, dans lequel $P^1$ est un groupe benzyle ou un groupe p-méthoxybenzyle.

**3.** Procédé selon la revendication 1 ou 2, dans lequel $P^3$ est un groupe benzyle ou un groupe alkyle en $C_1$ à $C_2$.

**4.** Procédé selon la revendication 1, dans lequel $P^1$ et $P^3$ sont tous deux des groupes benzyle.

**5.** Procédé selon l'une quelconque des revendications 1 à 4, dans lequel la position de liaison du groupe hydroxy sur le cycle benzène dans la formule (5) est la position 4 ou la position 3, ou dans lequel les positions de liaison pour le groupe hydroxy et le groupe -Y sur le cycle benzène dans la formule (5) sont de préférence adjacentes l'une à l'autre.

**6.** Procédé selon l'une quelconque des revendications 1 à 4, dans lequel, dans la formule (5), la position de liaison du groupe hydroxy sur le cycle benzène est la position 4, et la position de liaison du groupe -Y sur le cycle benzène est la

position 3.

**7.** Procédé selon l'une quelconque des revendications 1 à 6, dans lequel le catalyseur au palladium devant être utilisé dans l'étape 3 est le [1,1'-bis(diphénylphosphino)ferrocène]dichloropalladium(II) ($PdCl_2$(dppf)), dans lequel la réaction de l'étape 3 est de préférence effectuée dans un solvant sulfoxyde ou un solvant amide, ou dans lequel la base devant être utilisée dans l'étape 3 est de préférence un acétate de métal alcalin.

**8.** Procédé selon l'une quelconque des revendications 1 à 7, dans lequel Y est un groupe boryle ($-B(OH)_2$) ou un groupe 4,4,5,5-tétraméthyl-1,3,2-dioxaboran-2-yle.

**9.** Composé représenté par la formule (5a) qui suit ou un sel de celui-ci ;

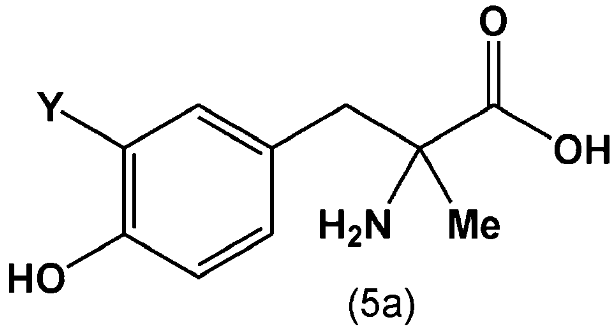

(5a)

dans lequel Y est un groupe boryle ($-B(OH)_2$) ou son groupe ester.

**10.** Composé représenté par la formule (4a) suivante ;

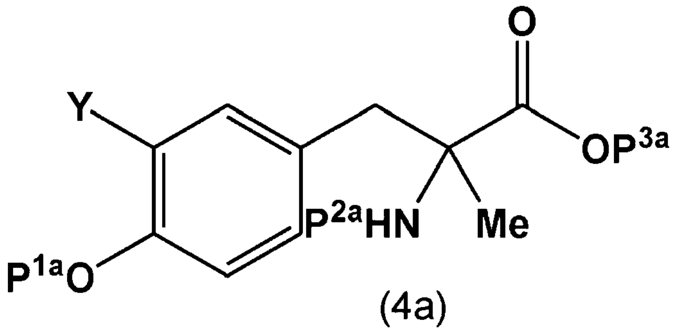

(4a)

dans lequel

$P^{1a}$ est un groupe benzyle ou un groupe p-méthoxybenzyle ;
$P^{2a}$ est un groupe tert-butoxycarbonyle ;
$P^{3a}$ est un groupe benzyle ou un groupe alkyle en $C_1$ à $C_2$ ; et
Y est un groupe boryle ($-B(OH)_2$) ou son groupe ester.

**11.** Procédé pour produire un composé radiomarqué représenté par la formule (6) ou un sel de celui-ci, comprenant l'étape 5 suivante ;

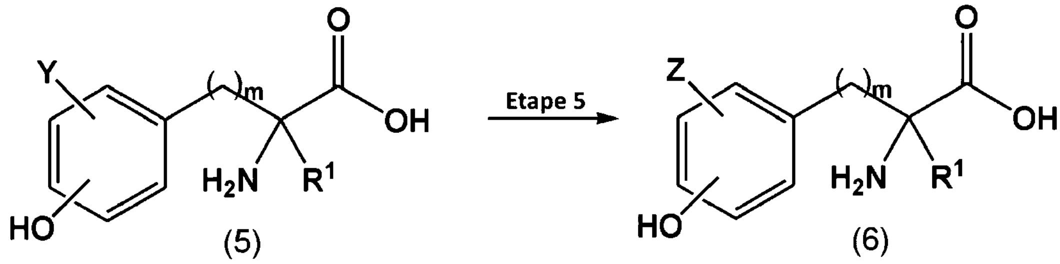


(5) (6)

dans lequel

$R^1$ est un atome d'hydrogène ou un groupe alkyle en $C_1$ à $C_4$ ;
m vaut 0, 1 ou 2 ;
Y est un groupe boryle (-B(OH)$_2$) ou son groupe ester ; et
Z est $^{211}At$, $^{210}At$, $^{123}I$, $^{124}I$, $^{125}I$ ou $^{131}I$,
étape 5 : une étape de réaction d'un composé représenté par la formule (5) ou d'un sel de celui-ci avec un radionucléide choisi parmi $^{211}At$, $^{210}At$, $^{123}I$, $^{124}I$, $^{125}I$ et $^{131}I$, en présence d'un réactif choisi parmi un iodure de métal alcalin, un bromure de métal alcalin, le N-bromosuccinimide, le N-chlorosuccinimide, le N-iodosuccinimide et le peroxyde d'hydrogène, dans de l'eau pour que soit obtenu le composé radiomarqué représenté par la formule (6) ou un sel de celui-ci.

**12.** Procédé selon la revendication 11, dans lequel le composé représenté par la formule (5) ou un sel de celui-ci est produit par un procédé tel que défini dans l'une quelconque des revendications 1 à 12 et/ou dans lequel la réaction est effectuée dans un système exempt de solvant organique, et/ou dans lequel la réaction est effectuée dans la plage allant de la température ambiante à 100°C, et/ou dans lequel le radionucléide est $^{211}At$ ou $^{131}I$, et le réactif est choisi parmi l'iodure de potassium et le N-bromosuccinimide, et/ou comprenant en outre une étape de purification du composé radiomarqué représenté par la formule (6) ou d'un sel de celui-ci, et/ou comprenant en outre une étape de stabilisation du composé radiomarqué représenté par la formule (6) ou d'un sel de celui-ci par addition d'acide ascorbique.

# Figure 1

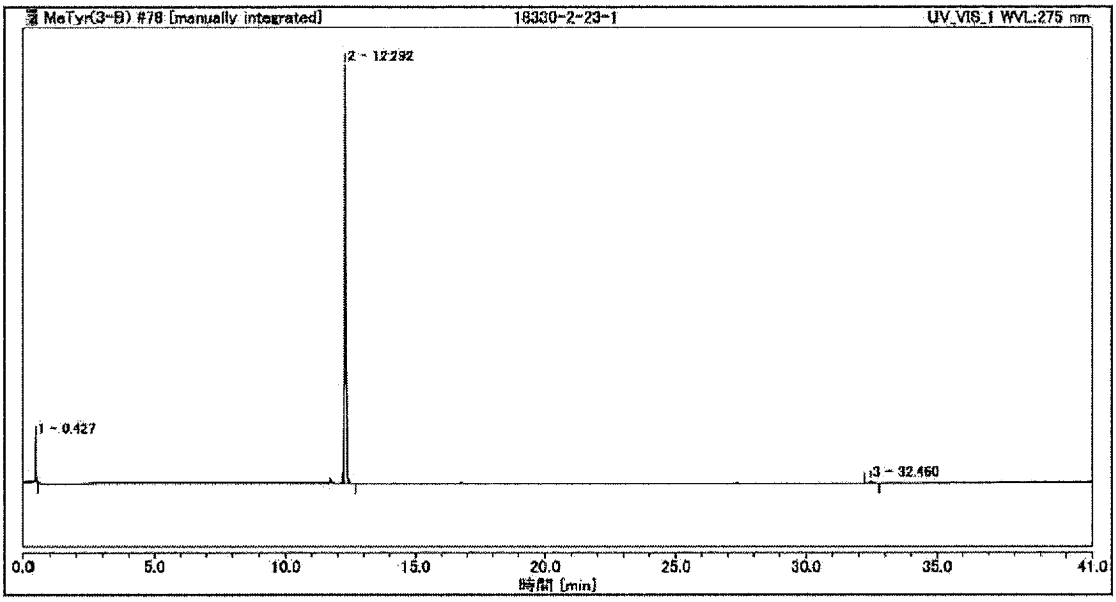


# Figure 2

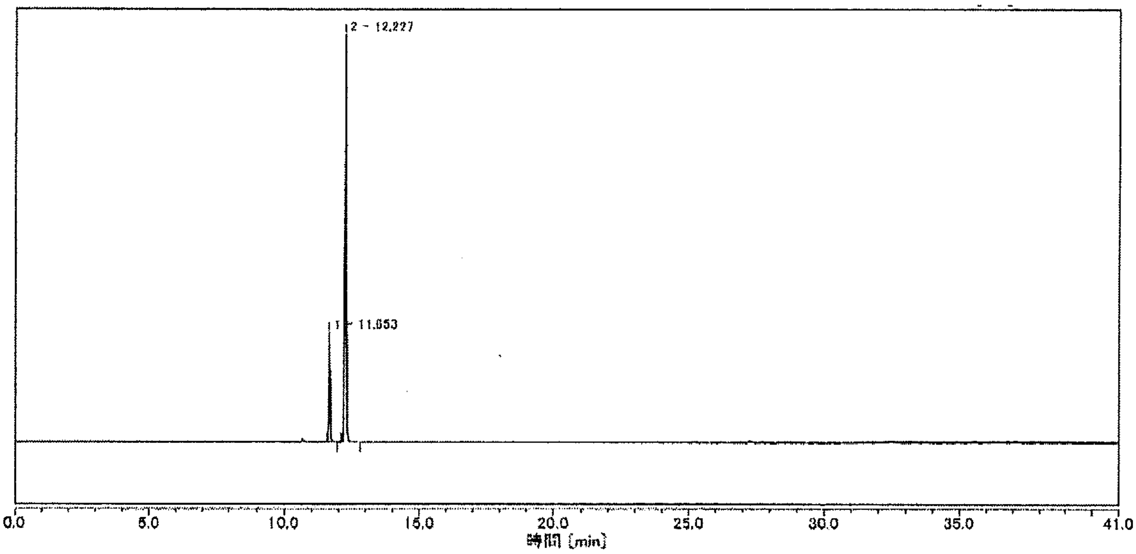

Figure 3

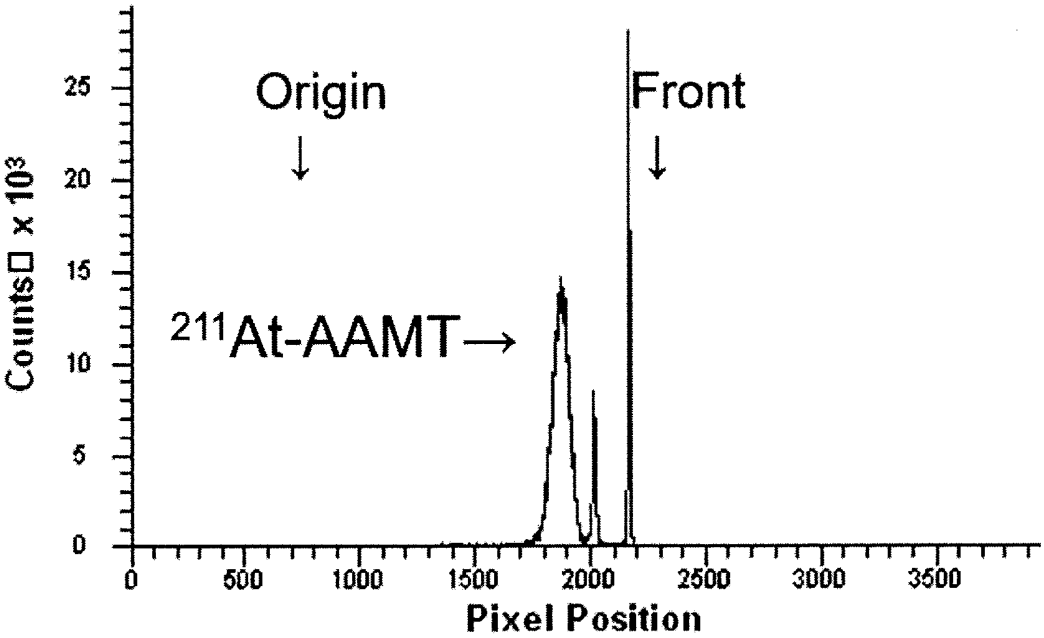


Figure 4

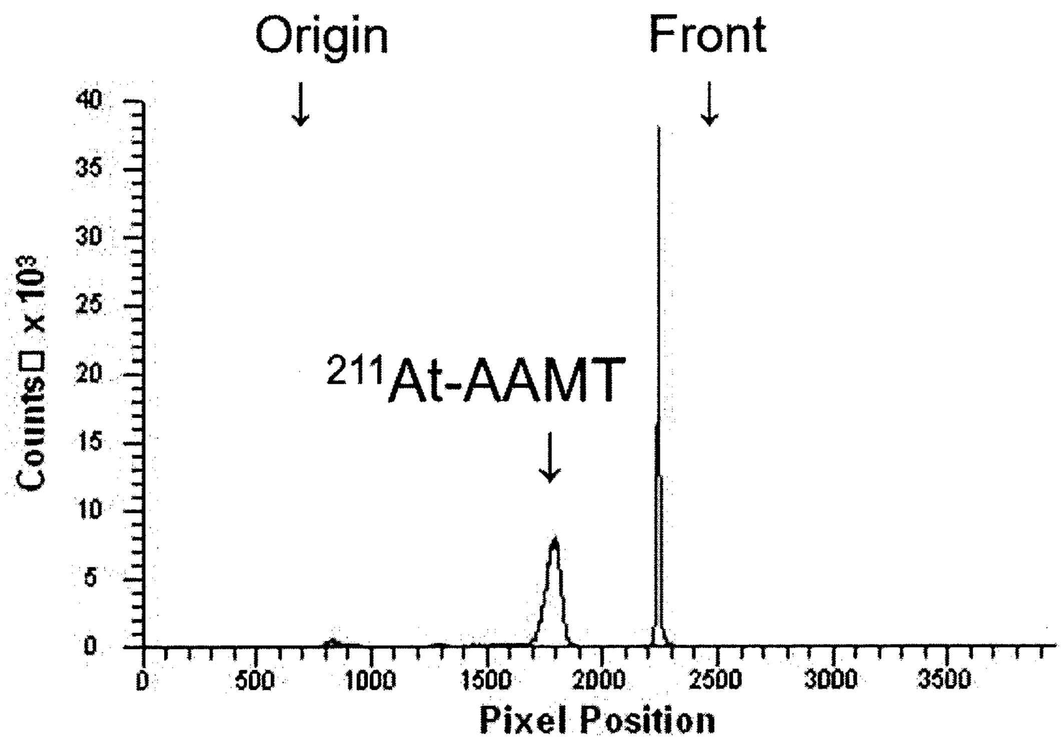

Figure 5

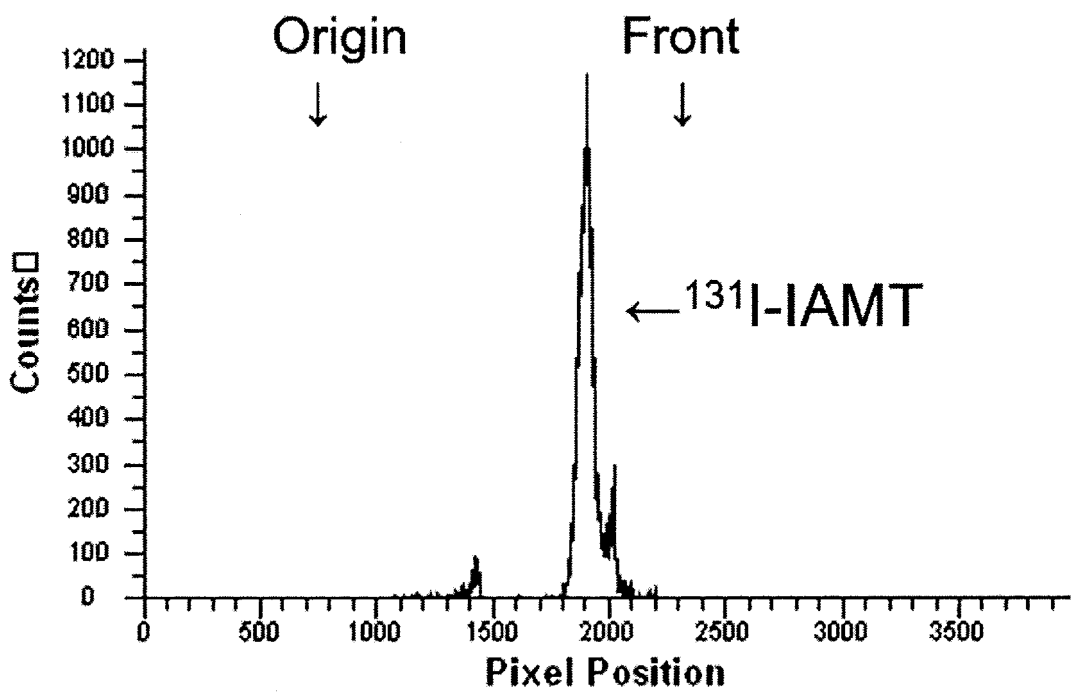
Origin
Front
$^{131}$I-IAMT
Counts
Pixel Position
0
100
200
300
400
500
600
700
800
900
1000
1100
1200
0
500
1000
1500
2000
2500
3000
3500

# Figure 6

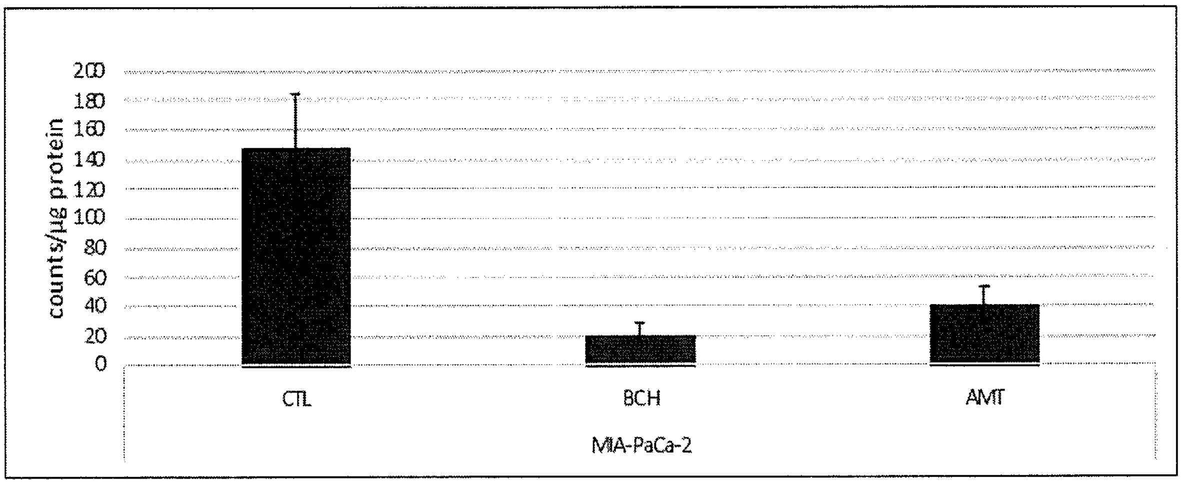


Figure 6a $^{211}$At-AAMT intracellular uptake
(Method of present invention)

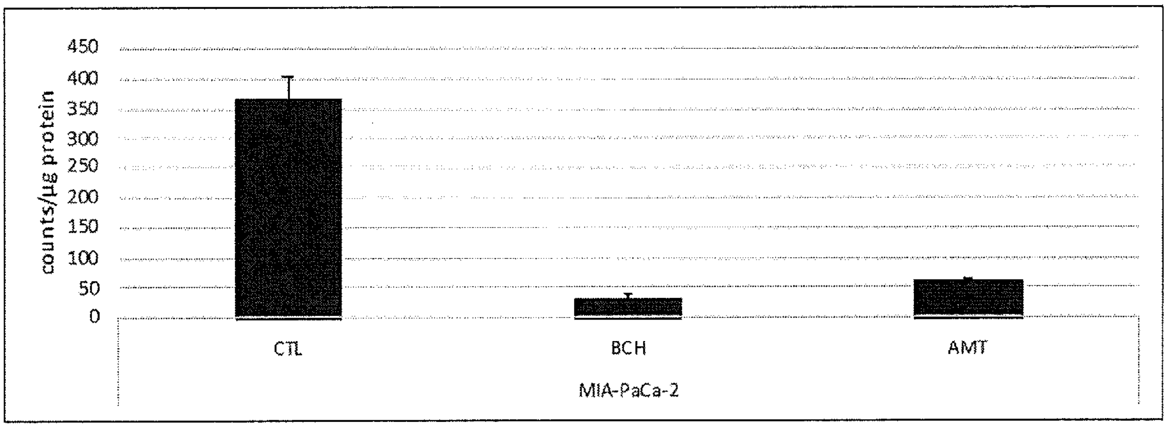


Figure 6b $^{211}$At-AAMT intracellular uptake
(Mercury method)

Figure 7

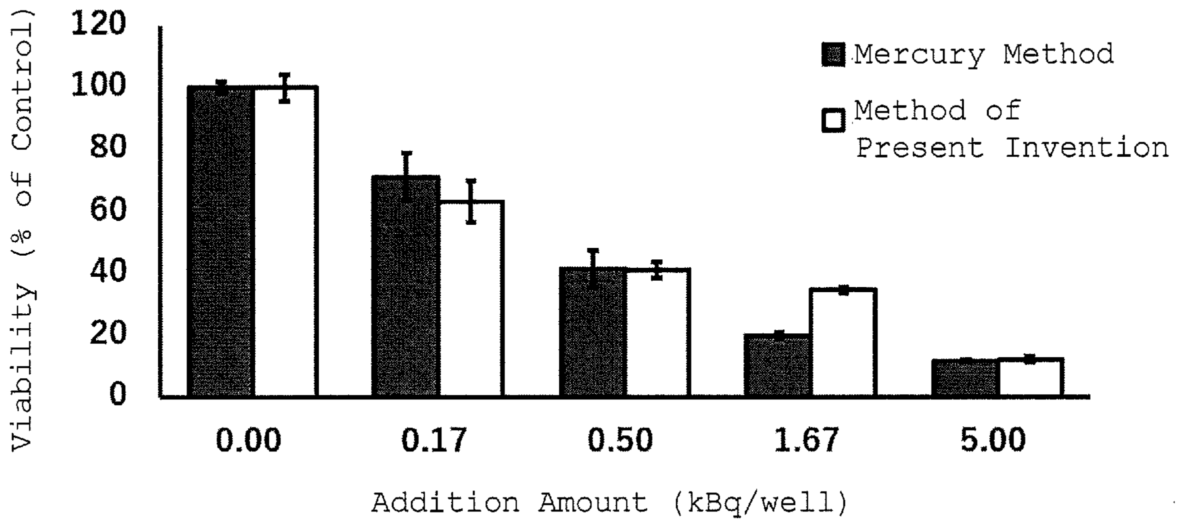
Viability (% of Control)
120
100
80
60
40
20
0
Mercury Method
Method of Present Invention
0.00
0.17
0.50
1.67
5.00
Addition Amount (kBq/well)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019176505 A
- WO 2019027059 A
- WO 2020180390 A
- US 2021000988 A


### Non-patent literature cited in the description


- *J Surg Oncol*, 1988, vol. 37, 192-7
- *Int J Appl Radiat Isotop*, 1979, vol. 30, 749-52
- **GHOSH SAMIR et al.** *ARKIVOC*, 2009, vol. 7, 72-78
- **VISSER et al.** *Int.J.Appl. Ratiation and Isotops*, 1979, vol. 99, 749-752